# EUROPEAN PATENT SPECIFICATION

(11) **EP 0 754 193 B1**
(45) Date of publication and mention of the grant of the patent: **14.06.2000**
(21) Application number: 95916141.5
(22) Date of filing: 07.04.1995
(51) Int. Cl.: C07K 14/18, A61K 39/29, G01N 33/68

(54) **HEPATITIS C VIRUS CORE PEPTIDE FOR STIMULATION OF CYTOTOXIC T LYMPHOCYTES AND DIAGNOSIS OF HCV EXPOSURE**
PEPTID AUS DEM INNEREN DES HEPATITIS-C-VIRUS BRAUCHBAR FÜR DIE STIMULATION CYTOTOXISCHER T-LYMPHOCYTEN UND DIE DIAGNOSE DES HCV-KONTAKTES
PEPTIDE DE NOYAU DU VIRUS DE L'HEPATITE C POUR LA STIMULATION DES LYMPHOCYTES T CYTOTOXIQUES ET LE DIAGNOSTIC DE L'EXPOSITION AU VHC

(30) Priority: 08.04.1994 US 224973
(43) Date of publication of application: 22.01.1997
(73) Proprietor: THE GOVERNMENT OF THE UNITED STATES OF AMERICA, represented by THE DEPARTMENT OF HEALTH & HUMAN SERVICES, Rockville, MD 20852 (US)
(72) Inventor: BERZOFSKY, Jay, A., Bethesda, MD 20814 (US); FEINSTONE, Stephen, Washington, DC 2008 (US); SHIRAI, Mutsunori, Kagawa 761-01 (JP)
(74) Representative: Desaix, Anne
(86) International application number: US9503935
(87) International publication number: WO9527733

(56) References cited:
- WO-A-94/20127
- WO-A-95/12677
- DE-A- 4 209 215
- JOURNAL OF VIROLOGY, vol. 68, no. 5, May 1994 pages 3334-3342, M. SHIRAI ET AL. 'An Epitope in Hepatitits C Virus Core Region Recognized by Cytotoxic T Cells in Mice and Humans'
- DATABASE WPI Section Ch, Week 9507 Derwent Publications Ltd., London, GB; Class B04, AN 95-047903 & JP-A-06 327 482 ( IMMUNO JAPAN KK) , 29 November 1994
- PROCEEDINGS OF THE NATIONAL ACADEMY OF SCIENCES OF USA, vol. 89, April 1992 WASHINGTON US, pages 3190-3194, W.-M. CHING ET AL. 'Interaction of immune sera with synthetic peptides corresponding to the structural protein region of hepatitis C virus'

## Description

### BACKGROUND OF THE INVENTION

### Field of the Invention

The present invention is directed to peptides derived from the core protein of the Hepatitis C Virus (HCV). The peptides are those which elicit a cytotoxic T lymphocyte (CTL) response in an immunized host. The invention is also directed to vaccines for prevention and treatment of HCV infection and diagnostic methods for detection of HCV exposure in patients.

### Description of Related Art

Hepatitis C virus is not only the cause of most cases of parenterally acquired non-A, non-B hepatitis, but also is responsible for a large portion of sporadic community acquired acute viral hepatitis, chronic hepatitis of unknown origin, cryptogenic cirrhosis and probably hepatocellular carcinoma (2,18,37,54). It is the propensity of this virus to cause chronic infections and chronic liver disease that makes it such a medically important problem. Therefore, there is an important need for a vaccine to protect against infection by this virus and diagnostic tests to assess exposure of patients to HCV.

HCV is a single-stranded plus sense RNA virus (10) and has been classified as a member of the Flaviviridae family (8,28). The structural proteins of the virus consist of the core, which forms the nucleocapsid, and two envelope glycoproteins E1 and E2. Because the envelope proteins are highly variable in sequence (33) and CTL clones may distinguish different isolates of HCV, as has been shown in HIV-1 studies (46,63,64), immunization with the envelope protein may not be an ideal approach for HCV. In contrast to the substantial amino acid sequence variation in the predicted envelope glycoproteins, the core protein of HCV shows greater sequence conservation among isolate groups (33) and is of particular interest for a vaccine to induce CTL. The conservation of core protein sequence also makes this protein a good target for a diagnostic method for assay of exposure to HCV based upon recognition of peptides of the HCV core protein by CTL in the exposed subject.

Like the related pestiviruses of animals, HCV infections may cause acute, self limited disease as well as chronic infections that result in chronic liver disease, cirrhosis and hepatocellular carcinoma. Neither the mechanism of chronicity nor the pathogenesis of the liver disease is understood. An immune escape mechanism has been proposed to account for the chronic infections based on a hypervariable region that has been identified within the E2 protein (29,70). Multiple sequences in this hypervariable region can be obtained from the same patient at the same time though one sequence usually predominates. Weiner et al. have suggested that the predominant sequence changes over time under immune selection, and that this hypervariable region is the major neutralization epitope of the virus (71). Experimental inoculations and challenge experiments in chimpanzees have also failed to demonstrate that these animals mount an effective protective antibody response following infection (23,53).

Therefore, it is important to define the T cell responses in HCV infections and to determine how they relate to immunity as well as pathogenesis. As T cell epitopes may be found in non-structural components of the virus and therefore may not be under the same immunologic pressure as the surface antigens, they may be important additions to a vaccine.

Cytotoxic T lymphocytes have been found to mediate protection *in vivo* against certain virus infections (19,51,52). The chronicity of infections as well as histopathologic findings indicate that HCV is probably not directly cytopathic (or cytolytic) in hepatocytes. Previous studies have reported that CD8⁺ CTL recognize epitopes within HCV proteins (38,60). The addition of CTL epitopes to a potential vaccine might overcome some of the problems apparent with vaccines produced from only the surface glycoproteins.

Class I and class II MHC molecules allow T cells to recognize polypeptide fragments of protein following processing of foreign antigens (3,26,56,59,66,73). In particular, class I MHC molecules sample and present peptides cleaved from endogenously synthesized proteins, including those of infecting viruses, allowing CD8+ CTL to carry out immune surveillance against virally infected cells. Therefore, any viral protein synthesized in the cell, even if it is not expressed intact on the cell surface, is a potential target for such CTL. Synthetic peptide vaccines are advantageous in that they may elicit fewer deleterious immune responses than a whole protein or attenuated or killed virus immunogen (4).

DE-A-42 09215 HCV core peptides and their use as vaccines. These HCV core peptides have biological activity as antibody epitopes, but not as CTL epitopes.

WO 94/20127 teaches various HLA-A2.1 binding peptides, but excludes those peptides having negative binding residues at positions 1, 3, 6 and/or 7 in the case of peptides which are nine amino acids in length, since they fail to be immunogenic; i.e., induce a cytotoxic T. lymphocyte response.

### SUMMARY OF THE INVENTION

The invention resides in part in peptides representing CTL epitopes of the core protein of HCV. The peptides are representative of those fragments of HCV presented on the surface of HCV infected cells bound to MHC molecules.

The peptides can be used both as an immunogen, as part of a vaccination protocol, or as a diagnostic or prognostic tool. In the former application, the peptides are formulated into vaccines and administered to a subject for the prevention or treatment of HCV infection. In the diagnostic and prognostic applications, the peptides can be contacted with immune cells from a patient. The response of the immune cells to the peptide is then assessed.

### BRIEF DESCRIPTION OF THE DRAWINGS

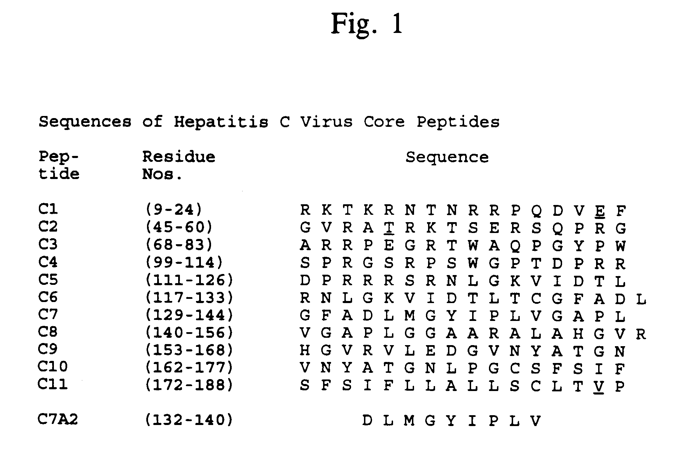

Figure 1 shows a series of 11 peptides selected by the amphipathicity algorithm for prediction of T cell epitopes from the amino acid sequence of the HCV core protein. The sequences of the synthesized peptides were based on the HCV-H isolate. Residues which differ from the published sequences (29,32,48,70) are shown with underlines.

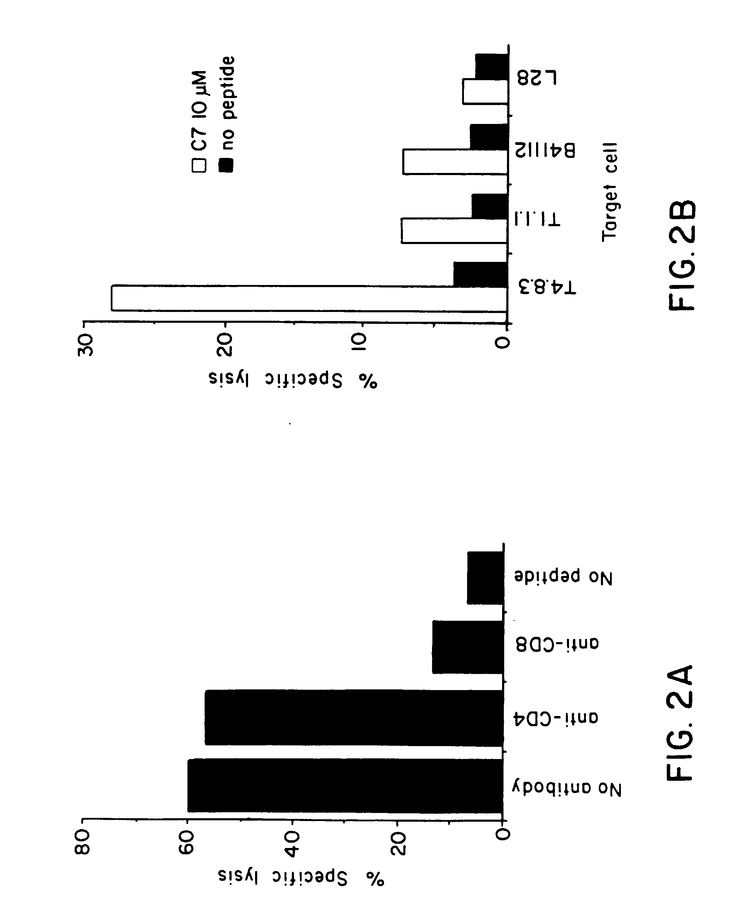

Figures 2A-2C show the results of tests of CTL specific for C7 for their phenotype, MHC-restriction, and fine specificity using overlapping peptides. Cells of a short-term CTL line derived from vHCV#4-immune spleen cells, restimulated with C7 at 10µM, together with irradiated syngeneic spleen cells, and a 1:10 dilution of the supernatant of a culture of spleen cells stimulated with concanavalin A (a source of cytokines), were used as effectors. Lysis in the absence of peptide was less than 7% in the experiments shown in Figs. 2A-2C. Target cells were sensitized in the presence of peptide (10µM). Effector/ Target ratio = 100/1. Data are the means of triplicate samples with an SE of less than 5% and are representative of at least two independent experiments.
**Fig. 2A; the phenotype of the H-2**^{**d**} **CTL specific for C7**. The CTL assay was performed in the presence of anti-L3T4 (GK 1.5) (anti-CD4) or anti-Lyt 2.2 (2.43) (anti-CD8) monoclonal antibodies (culture supernatant) at the dilution of 1:4, or no antibody, for 6 h. 18Neo was sensitized in the presence of C7 (10µM).
**Fig. 2B; the MHC class I molecules responsible for the presentation of C7 to CTL in the H-2**^{**d**} **strain**. Each transfectant expresses only one class I molecule from H-2d (T4.8.3, D^{d}; T1.1.1, L^{d}; and B4III2, K^{d} ) or none (L28). The parent cell in each case is a DAP3 L cell (H-2^{k}).

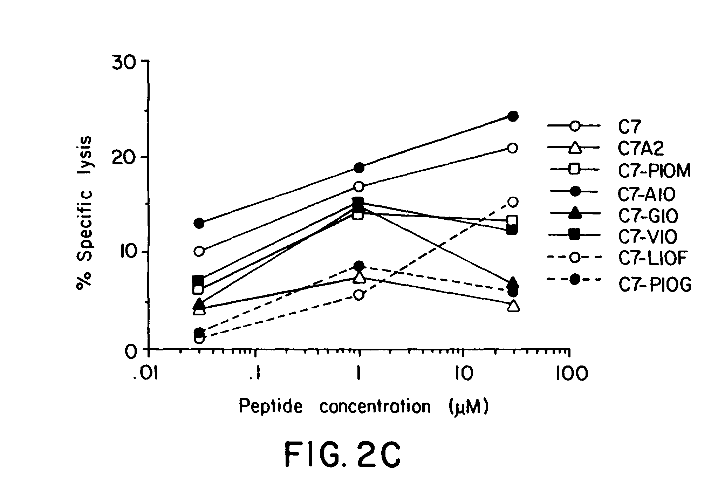

**Fig. 2C; fine specificity of murine H-2D**^{**d**}**-restricted CTL specific for C7**. CTL activity was tested on H-2^{d} 3T3 fibroblast target cell line 18Neo in the presence of titrated concentrations of the following overlapping decapeptides, SEQ. ID. NOS: 2-8, respectively contained in C7, as well as the HLA-A2 motif nonapeptide C7A2 (see Fig. 1) and the full-length C7 peptide:

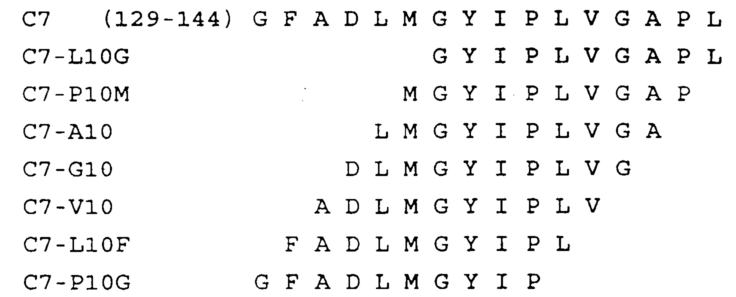

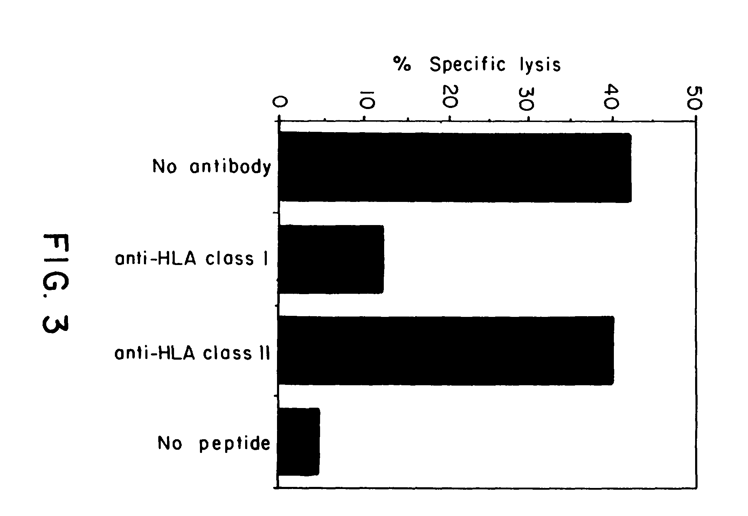

Figure 3 shows the HLA restriction of human CTL specific for C7 in a patient with HCV infection. Human CTL activity specific for C7 from a patient with chronic hepatitis C (No.7 in Table 4) tested for the cytotoxicity against the autologous target cell in the presence of C7 10µM with anti-HLA class I (W6/32, IgG2a) or anti-HLA class II DR (L-243, IgG2a) at 1:4 dilution or no antibody. The PBL were stimulated twice with mitomycin C-treated PBL and peptide C7 as described in "Material and Methods". The lysis in the absence of peptide is less than 5%. Data are the means of triplicate samples with an SE of less than 5% and are representative of at least two independent experiments.

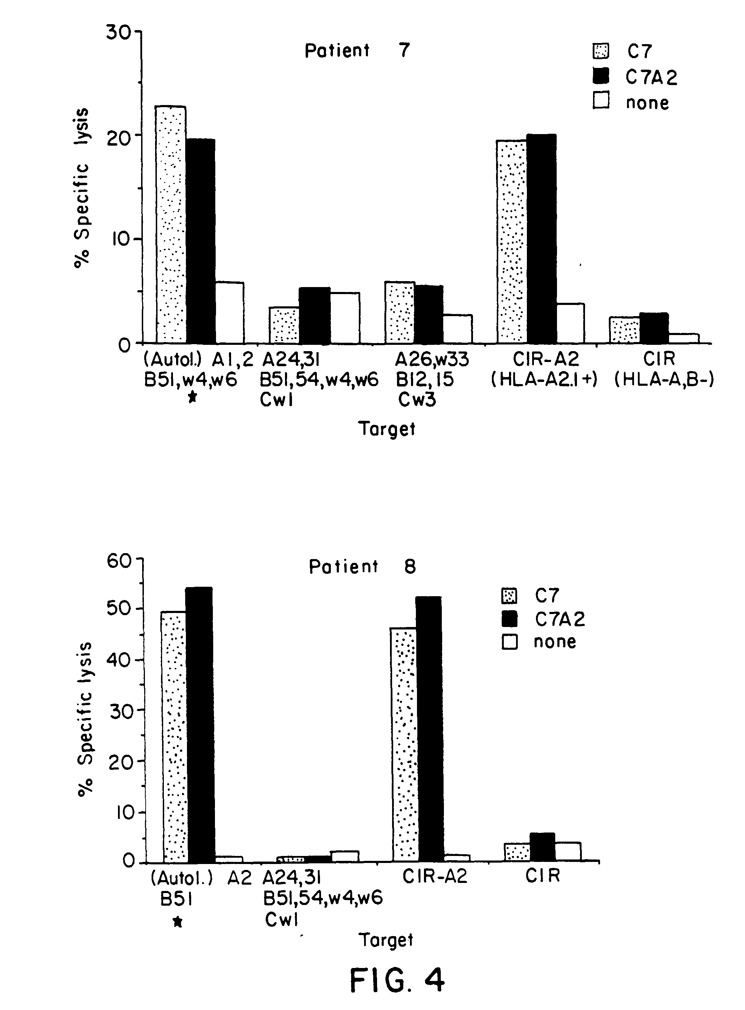

Figure 4 presents data showing that Human CTL recognize a nonamer segment of C7 presented by HLA-A2. PBL from patient #7 with chronic hepatitis C and #8 with acute hepatitis C (Table 4) were stimulated twice *in vitro* with mitomycin C-treated autologous PBL and peptide C7 as described in Materials and Methods, and tested at an effector-to-target ratio of 100: 1 in the presence of peptide C7, the nonamer peptide C7A2 (DLMGYIPLV), or no peptide against the following targets: autologous EBV-transformed B lymphoblastoid cells (HLA-A1,A2, B51, Bw4, Bw6 for #7) or autologous Con A blast targets for #8 (A2, B51 for #8) (autologous cells indicated by *), allogeneic EBV-transformed B lymphoblastoid cells (HLA-A24,31,B51,54,w4,w6,Cw1; or HLA-A26,w33, B12,15, Cw3), the C1R cell line (39) (HLA-A-neg, B-neg., Cw4, DR8, DPw4, DQ3) either transfected with HLA-A2.1 (ClR-A2) (39) or untransfected (ClR). Data are the means of triplicate cultures with all SEM < 5%, and are representative of at least two independent experiments.

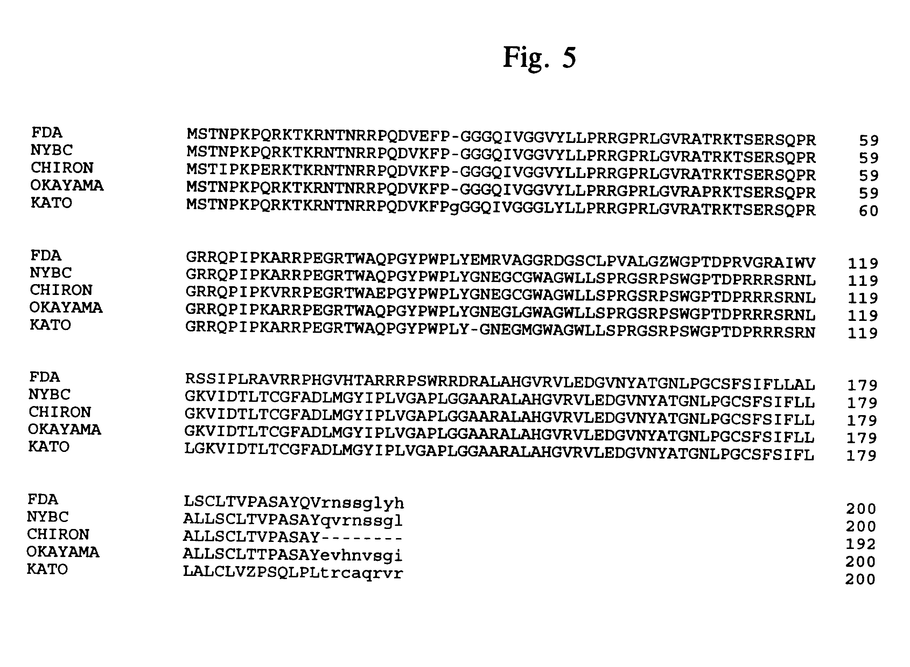

Figure 5 shows an alignment of HCV core protein sequences resident in GENBANK on April 5, 1994 and the HCV-H sequence used in the working examples, SEQ. ID. NOS. 22-26. Amino acid residues in bold indicate residues that vary among sequences.

### DETAILED DESCRIPTION OF THE INVENTION

The peptides of the present invention have an amino acid sequence derived from the sequence of the core protein of the HCV virion. Several isolates of HCV have been obtained and the amino acid sequence of the core protein was found to be 98-99% identical among all of them (33). The amino acid sequences of core protein from several HCV isolates is presented in Figure 5. The HCV-H isolate, used for the present working examples, is shown on the top line (FDA). The alignment shows that the carboxyl terminus of the core protein diverges significantly among the isolates sequenced.

As described in more detail in the Examples, the conservation of the core protein sequence is advantageous with respect to design of a peptide vaccine and diagnostic reagent. The CTL elicited in response to immunization with peptides of the present invention attack infected cells rather than free virions. Because fragments of all of the proteins endogenously synthesized by a cell are displayed on the surface of the cell, bound to MHC molecules, the fact that the core protein is not localized to the surface of the virion particle is not problematic.

Furthermore, the mechanism of processing of endogenous proteins for display by the MHC is such that it is expected that any collinear peptide that can be obtained by random proteolysis of a protein is likely to be generated at early steps in the processing. The particular MHC complexes expressed by an individual appear to be responsible for selection of the particular peptides that are actually found on the surface of cells of the individual (26). Thus, a person having an HLA-A11 haplotype will likely display different peptides from a particular protein than are displayed by a person having an HLA-A2 haplotype.

The particular rules explaining selection of peptides that bind to particular MHC haplotypes are not fully defined. However, what is known of the structure-binding relationship has been summarized in "motifs" for peptide binding to MHC haplotypes. A table of motifs published in the literature to date has been compiled by Gabriel Meister and Dr. Anne DeGroot at Brown University (Providence, RI) and is presented as Table 1.

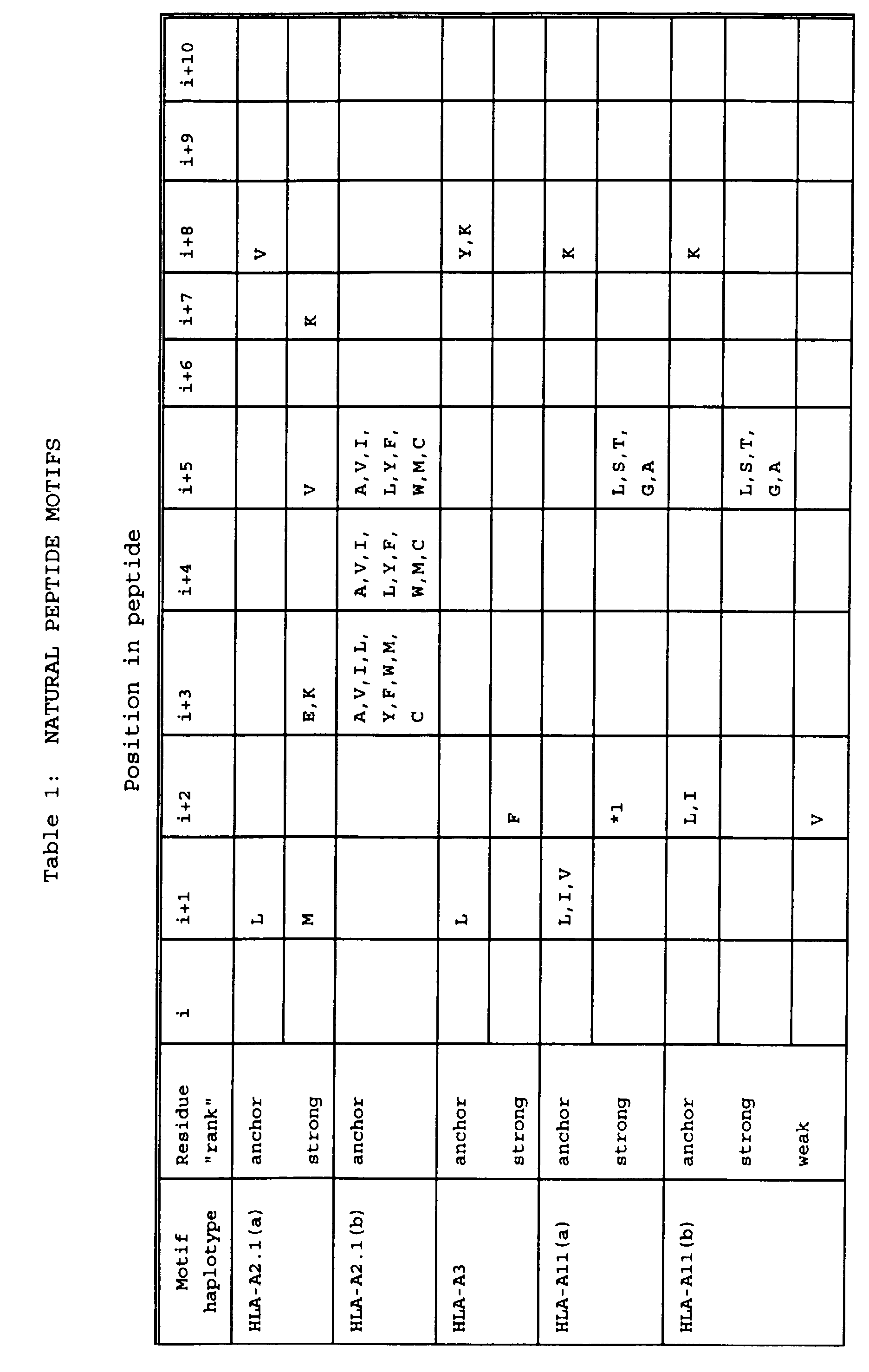

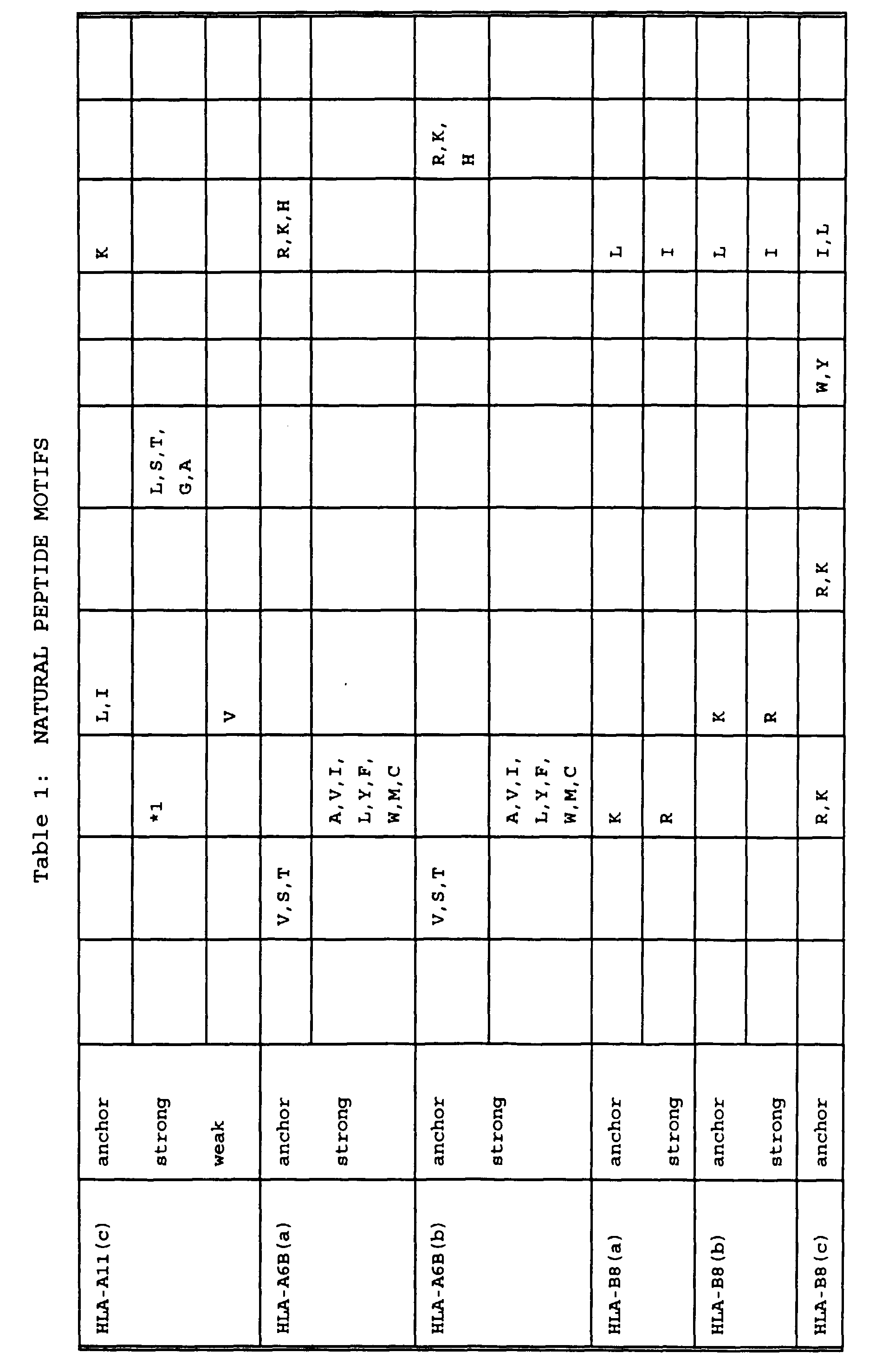

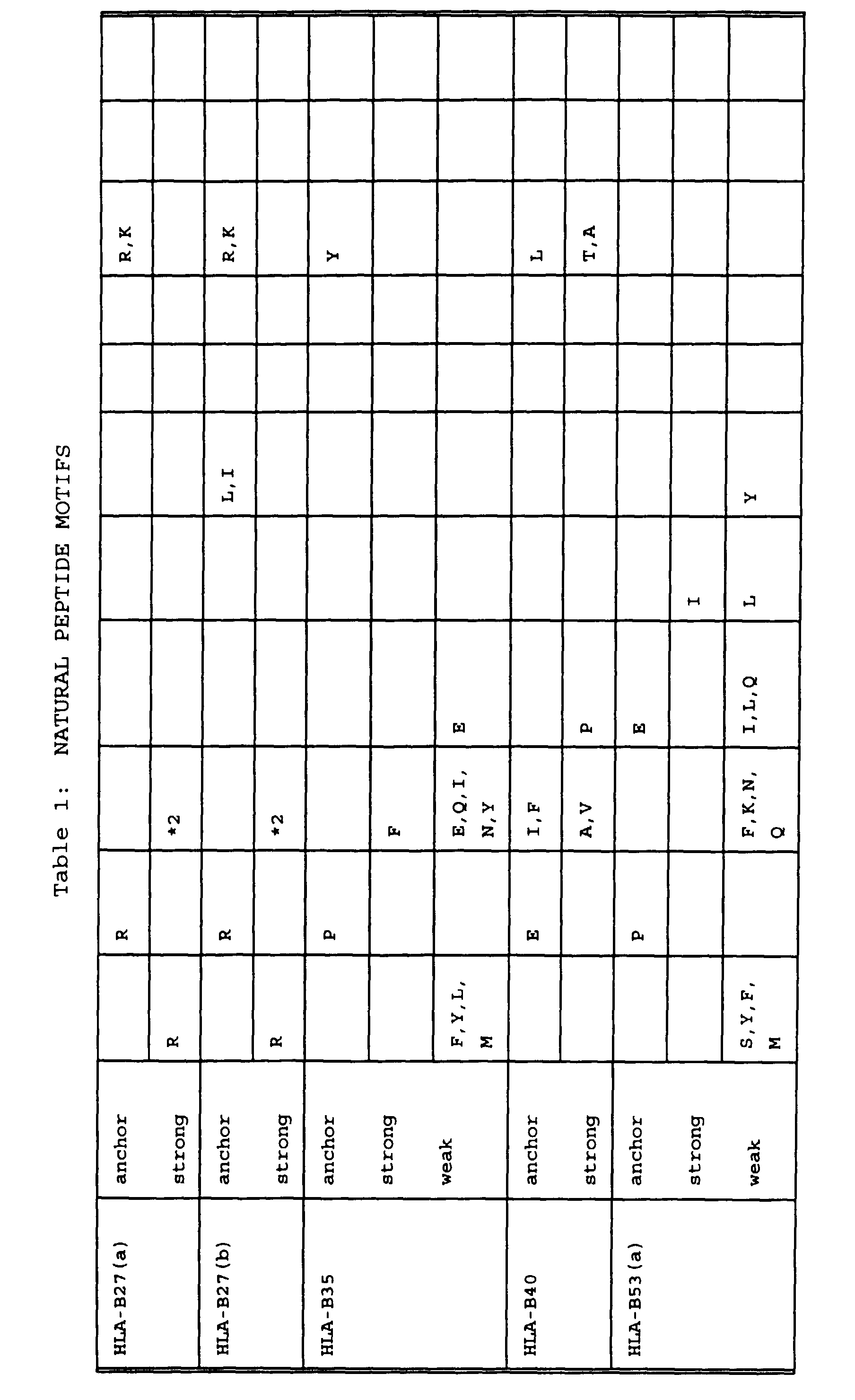

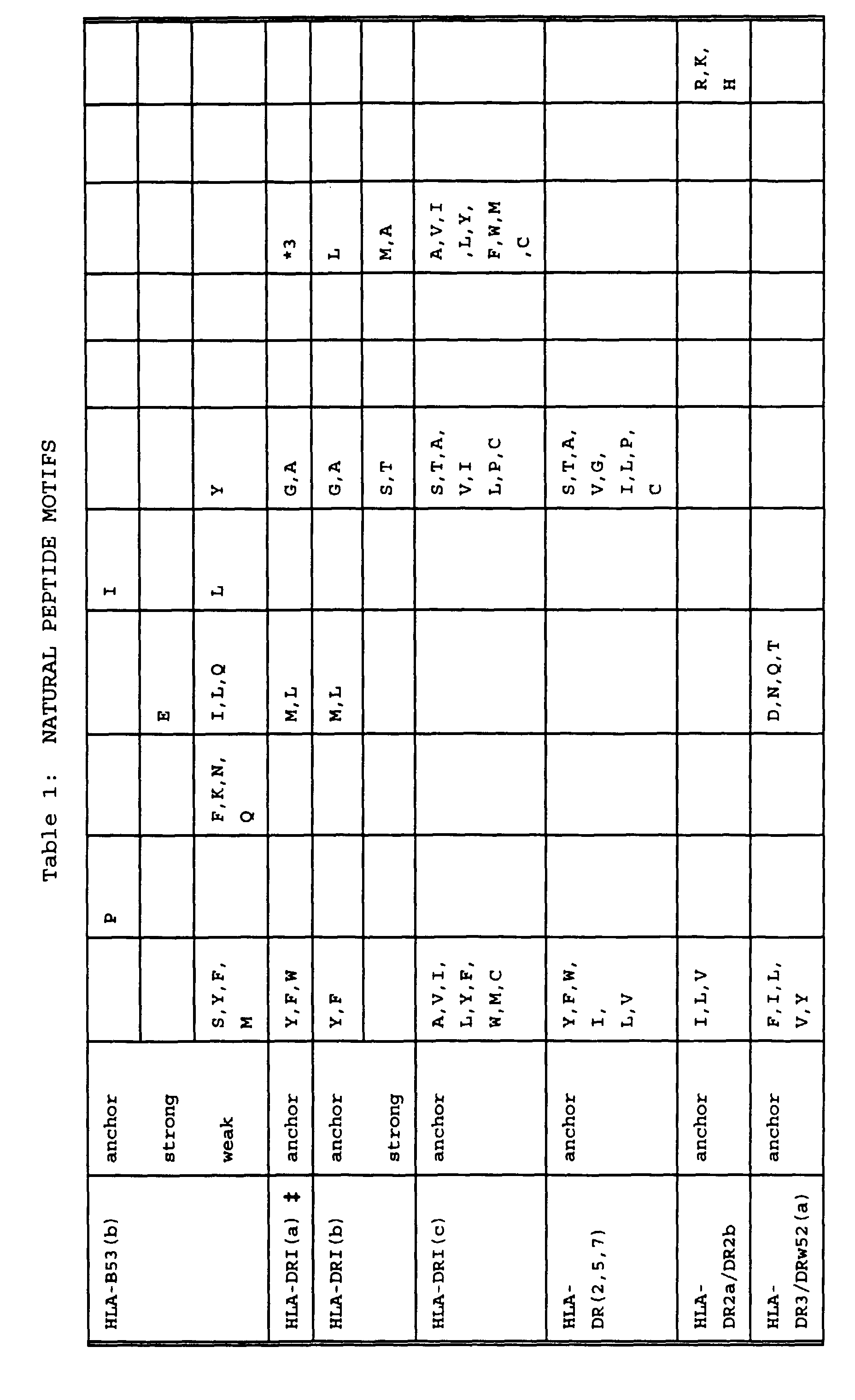

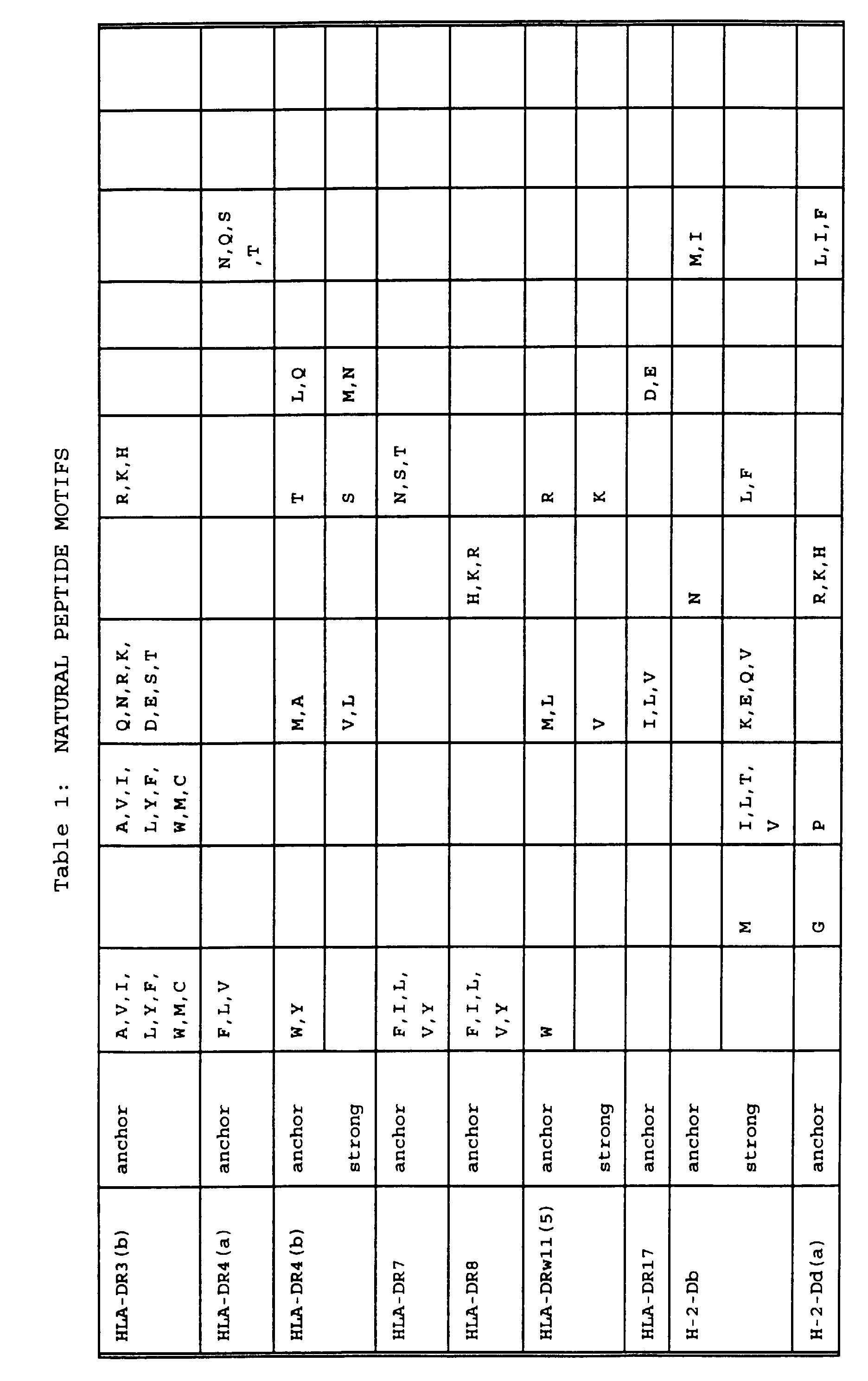

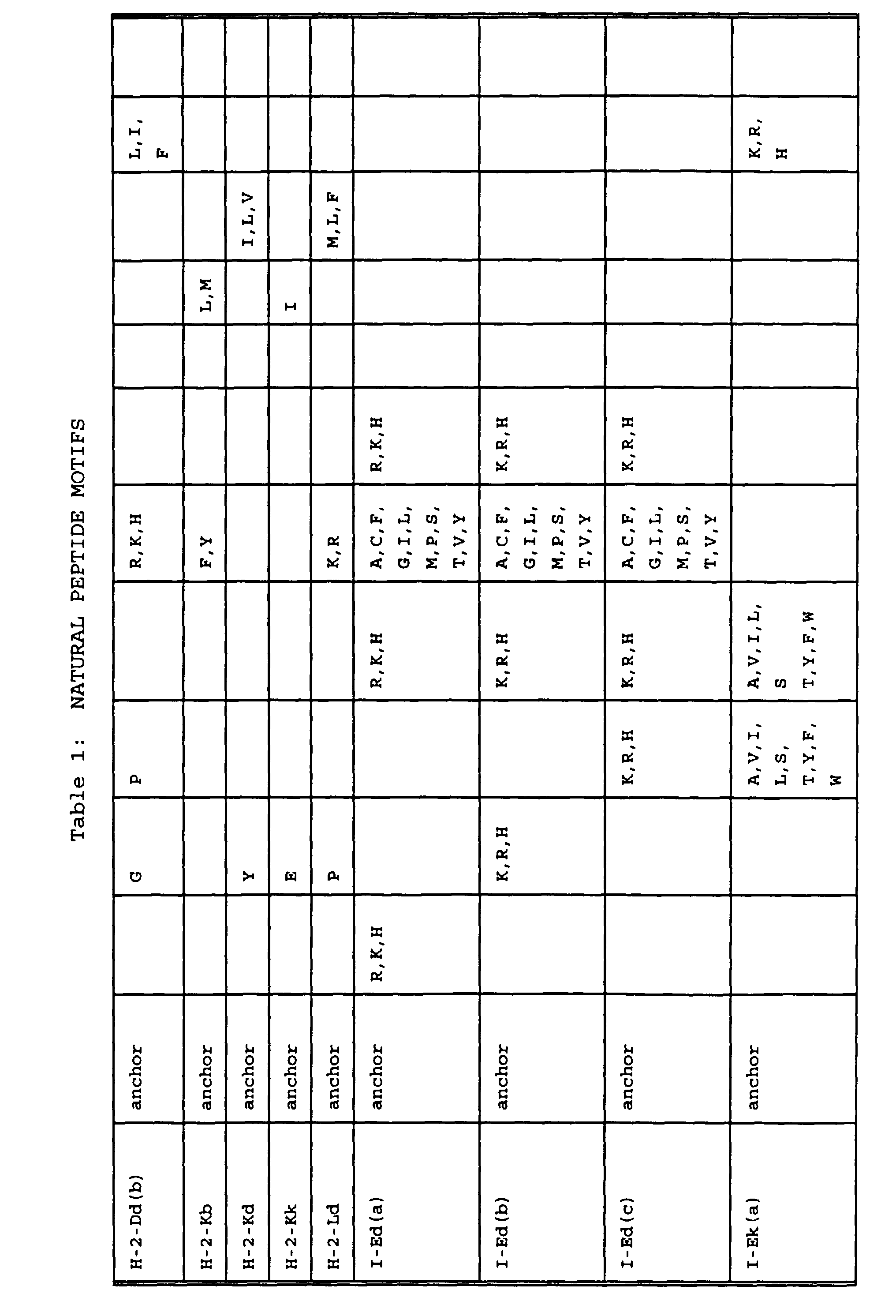

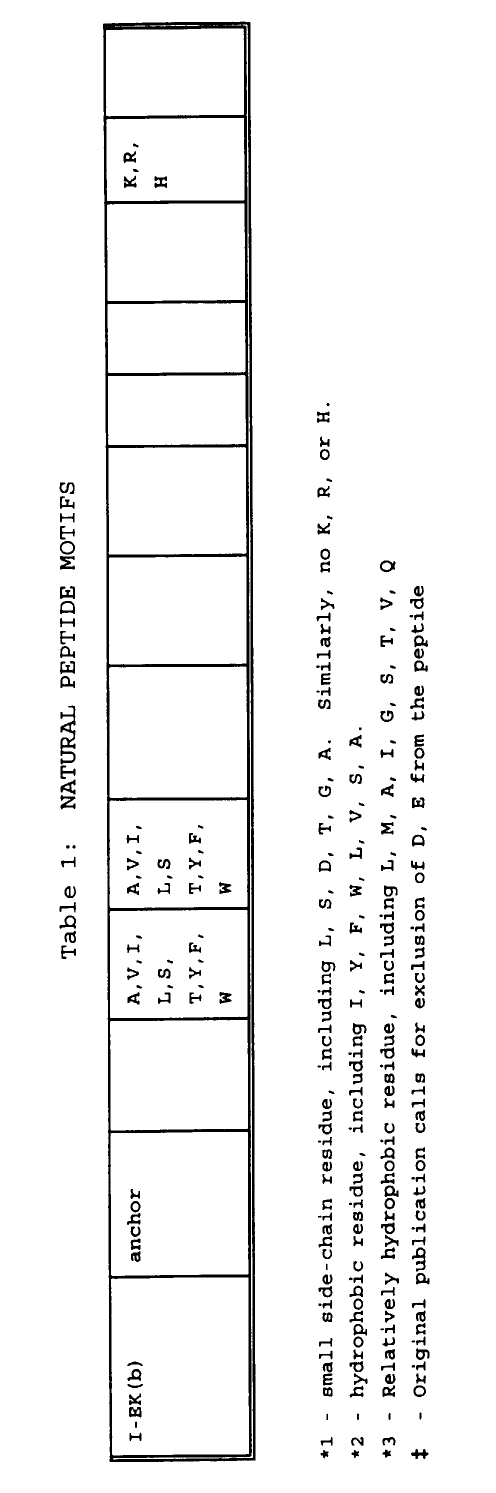

As described below, the C7 peptide was found first by actual testing for CTL activity and was later found to have a sequence consistent with the HLA-A2 binding motif. The C7 peptide is expected to be useful in only the population which has the HLA-A2 haplotype. However, since this is 40-50% of the human population, this is a significant population.

Furthermore, peptides containing binding motifs for additional HLA types are identifiable within the peptides used in Examples 1 and 2. For example the C1, C2, C4, C6 and C8 peptides (see Figure 1) have binding motifs for human HLA-A68, HLA-A68, HLA-A68, HLA-B8, and HLA-B27, respectively. To determine the usefulness of these peptides, each is tested for CTL activity, a positive result confirming that the particular peptide is effective for eliciting a CTL response and can be used as a diagnostic reagent for subjects having the corresponding MHC (HLA in human subjects) haplotype.

### General Experimental Materials and Methods

**Mice**. BALB/c mice and C57BL6 mice were purchased from Japan Charles River Laboratories (Tokyo, Japan). Mice used were 8 weeks old.

**Recombinant Vaccinia Viruses**. Recombinant vaccinia virus expressing the HCV structural genes C, E1, and E2, as well as NS2 (FDA isolate of the H strain HCV(H) (24)) (vHCV#4) was made by the method of Chakrabarti et al. (9) and used for immunizing the mice to generate HCV core specific CTL. vSC8 (recombinant vaccinia virus containing the Escherichia coli lacZ gene), a generous gift of Dr. Bernard Moss, NIAID, NIH, has been described (9) and was used as a control vaccinia for immunizing the mice.

**Peptide Synthesis and Purification**. HCV core peptides according to the predicted amino acid sequence of HCV(H) (24) (unpublished) were prepared by the simultaneous multiple peptide method of solid-phase peptide synthesis, in polypropylene mesh "tea-bags" as described (31). Peptides were desalted by reverse-phase chromatography on C18 Sep-Pak columns (Waters Associates, Milford, MA), purified and analyzed by HPLC.

**CTL Generation**. Mice were immunized intravenously with 10⁷ PFU of recombinant vaccinia virus. 4-6 weeks later, immune spleen cells (5x10⁶/ml) in 24-well culture plates in complete T cell medium (CTM; 1:1 mixture of RPMI 1640 and EHAA medium containing 10% FCS, 2mM L-glutamine, 100 U/ml penicillin, 100 µg/ml streptomycin and 5x10⁻⁵ M 2-ME) were stimulated for 6 days *in vitro* with peptides or vHCV#4-infected (1 hour, 37°C, multiplicity of infection of 10:1) irradiated syngeneic spleen cells (2.5x10⁶/ml well, three washings before culture) and 10% Con A supernatant-containing medium (Rat T Stim; Collaborative Research, Inc., Bedford, MA), and restimulated with irradiated syngeneic spleen cells (2.5x10⁶/ml well) and peptides at day 7, and addition of 10% rat T stim and replacement of 0.5 ml culture medium by fresh CTM at day 8 and 11. At day 7 or 14 of the culture, the stimulated cells were used as effectors in a CTL assay.

**CTL Assay**. Cytolytic activity of in vitro secondary CTL was measured as previously described (62, 67) using a 6-hour assay with ⁵¹Cr-labeled targets. For testing peptide specificity of CTL, effectors and ⁵¹Cr-labeled targets were mixed with various concentrations of peptide. The percent specific ⁵¹Cr release was calculated as 100 x [(experimental release - spontaneous release)/(maximum release - spontaneous release)]. Maximum release was determined from supernatants of cells that were lysed by addition of 5% Triton-X 100. Spontaneous release was determined from targets cells incubated without added effector cells. The 18Neo (H-2^{d}; class I MHC⁺, class II MHC⁻ neomycin-resistance gene-transfected 3T3 fibroblast (62)) and EL4 thymoma cells (H-2^{b}) were used as targets.

**Blocking of CTL response by antibodies**. Culture supernatant of hybridomas GK1.5 or 2.43 containing anti-L3T4 (anti-CD4, IgG2b (72) or anti-Lyt 2.2 (anti-CD8 (58)) antibodies, respectively, were added to the 96 well plates of CTL assay, at the indicated concentrations.

**Class I MHC transfectants**. Mouse L cell transfectants with D^{d}, L^{d} (21,41,43,45) were the kind gifts of Dr. David Margulies, NIAID. The transfectant expressing K^{d} was developed by Abastado et al. (1) and was a kind gift of Dr. Keiko Ozato (NICHD). All transfectant cell lines were examined by FACS analysis with an appropriate panel of anti-H-2D^{d}, anti-H-2K^{d}, and anti-H-2L^{d} mAbs to confirm their expressed phenotype before the performance of the functional studies reported here. The human C1R cell line (39) (HLA-A-neg, B-neg., Cw4, DR8, DPw4, DQ3) either untransfected (ClR) or transfected with HLA-A2.1 (ClR-A2) (39) was a kind gift of Dr. Victor Engelhard, University of Virginia.

### Example 1: Identification Of A CTL Epitope In The Core Protein Of HCV

Based on the predicted amino acid sequence of the core protein of the HCV-H isolate, a series of 11 overlapping peptides covering 72.3% of the HCV core sequence and selected on the basis of amphiphathicity (13,17,40) as potential T-cell epitopes, were synthesized. The FORTRAN source code of the computer program used for the selection has been published as an appendix to reference 75. Comparison of the predicted amino acid sequence of the core region of HCV-H to other published isolates (32,36,65) showed that three of the peptides had substitutions in one or two residues compared to the published sequence (Fig.1). To generate CTL specific for the peptides in mice, the spleen cells of mice immunized with the recombinant vaccinia virus were stimulated *in vitro* with peptides. BALB/c mice that were immunized with vHCV#4 developed CTL responses to peptide C7 but not to any of the other peptides (Table 2). H-2^{b} mice showed no response to any peptide tested.

**Table 2.**

| CTL response to peptides from HCV core in H-2^{d} (BALB/c) mice and H-2^{b} (C57BL6) mice. | | |
|---|---|---|
| Peptide | % specific ⁵¹Cr release | |
| | H-2^{d} | H-2^{b} |
| | | |
| 1 | 4.7 | -0.2 |
| 2 | 3.2 | -1.1 |
| 3 | 3.2 | 0.0 |
| 4 | 3.2 | -1.3 |
| 5 | 3.6 | 2.0 |
| 6 | 3.9 | 0.3 |
| 7 | 26.3 | 0.0 |
| 8 | 4.1 | 0.6 |
| 9 | 2.8 | 2.0 |
| 10 | 4.9 | 1.4 |
| 11 | 2.5 | 1.3 |

Mice were primed i.v. with 10⁷ plaque-forming units of recombinant vacdnia virus expressing the HCV-H core region (vHCV#4). The immune spleen cells were restimulated in vitro with peptides at 10 µM or no peptide in the presence of Con A supernatant (IL-2) for 13 days as described in "Material and Methods". CTL activity was measured against neo gene transfected 3T3 fibroblast cells (18Neo, H-2^{d} class I positive, class II negative) for BALB/c CTL and EL4 (H-2^{b}) for C57BL/6 CTL. Targets were sensitized with 10 µM of each peptide or no peptide for 6h. Effector / target (E:T) ratio 100:1, 5000 target cells/well. The lysis in the absence of peptide was <5% in BALB/c and C57BL/6. Data are the means of triplicate samples with an SE of < 5% and are representative of at least two independent experiments.

Specificity of CTL for the core protein was demonstrated at the level of lymphocyte priming *in vivo,* restimulation *in vitro,* and expression on the target cells in the CTL assay (Table 3). Only the recombinant vaccinia virus expressing the core gene (vHCV#4), but not the control vaccinia viruses (vSC8), could prime mice for development of CTL specific for C7 (Table 3). The titration of the peptide concentrations used for stimulation of immunized spleen cells demonstrated that C7 was required at a concentration of 1 to 10 µM peptide for the stimulation of immune spleen cells to elicit the significant killing against H-2-matched target cells.

Because the CTL were generated by *in vitro* stimulation with peptide, it was important to confirm that they recognized the processed products of endogenously synthesized core protein, not just peptide. The CTL restricted by H-2^{d} (BALB/c) were able to kill the vHCV#4-infected syngeneic target cells (18Neo cells, BALB/c 3T3 fibroblasts transfected with the neomycin resistance gene) endogenously expressing core, as well as 18Neo cells in the presence of C7, but not the control targets, 18Neo infected with vSC8 (control vaccinia virus) or 18Neo in the absence of C7 (Table 3). Therefore, these CTL were specific for processed products of endogenously synthesized HCV core protein, not only for exogenous peptide.

Treatment of the CTL specific for C7 with anti-CD8 monoclonal antibody reduced or abrogated cytotoxic

**TABLE 3.**

| Priming and boosting requirements for induction of CTL specific for C7 in H-2^{d} mice | | | | | | |
|---|---|---|---|---|---|---|
| Immunization | Restimulation | % Specific lysis | | | | |
| | | vHCV#4 | vSC8 | C7 | C8 | no peptide |
| none | C7 | -2.8 | 1.6 | 0.5 | | 1.4 |
| vSC8 | C7 | 1.3 | 2.6 | 3.6 | | 4.2 |
| | vHCV#4 | 70.6 | 64.4 | 3.9 | | 4.7 |
| | vSC8 | 68.8 | 64.3 | 3.2 | | 4.8 |
| vHCV#4 | C7 | 17.7 | 4.8 | 24.8 | 2.3 | 3.6 |
| | *C7 | 26.0 | 6.2 | 60.2 | 6.4 | 6.8 |
| | C7 1µM | 12.6 | 3.9 | 14.3 | | 2.1 |
| | C7 0.1µM | 2.0 | 1.2 | 1.3 | | 0.2 |
| | vSC8 | 65.8 | 72.7 | 2.3 | | -0.1 |
| | C8 | 2.2 | 1.7 | 1.0 | 1.7 | 0.6 |

| | | | | | | |
|---|---|---|---|---|---|---|
| The ability of recombinant vaccinia viruses to prime and stimulate CTL specific for the products of inserted viral genes was used to generate CTL specific for HCV core in BALB/c (H-2^{d}) mice. Non-immune or immune spleen cells were restimulated in vitro with C7 10µM (or at the indicated concentrations) or vaccinia (vSC8) infected irradiated syngeneic spleen cells, and tested against vaccinia virus-infected 18Neo (1 h, 37°C, multiplicity of infection of 10:1, three washings before use) and 18Neo in the presence of the peptides (C7 10 µM; C8 10 µM) or no peptide at an E/T ratio of 100:1. vHCV#4, recombinant vaccinia virus expressing core & envelope of HCV-H; vSC8, control vaccinia virus; 18Neo, BALB/c 3T3 fibroblast, H-2^{d}. Data are the means of triplicate samples with an SE of < 5% and are representative of at least two independent experiments. *vHCV#4 immune spleen cells, restimulated twice with C7 10µM and 10% ConA supernatant as described in "Material and Methods" and ref. (61). | | | | | | |

activity on target cells, whereas anti-CD4 antibody did not (Fig. 2A). These data indicate that the effector cells which recognize C7 are conventional CD8⁺CD4⁻ (Lyt2⁺L3T4⁻ ) CTL. For H-2^{d} -restricted peptide specific CTL in BALB/c, 18Neo cells expressing class I but not class II MHC gene products were used as targets. These facts, plus the MHC restriction to H-2^{d}, not H-2^{b}, indicated that these CTL are class I MHC restricted, as expected for Lyt2⁺ CD8⁺ effector T cells.

We used transfectants expressing D^{d}, L^{d}, or K^{d} molecules to determine which molecule was specifically required for the presentation of C7 in H-2^{d}. The targets were labeled with ⁵¹Cr and cocultured with the effector cells in the presence of peptide. T4.8.3 (D^{d}) was found to present C7 (Fig.2b), whereas neither L^{d} nor K^{d} presented C7 to the CTL. Therefore, D^{d} was necessary and sufficient to present this peptide.

In a titration study, C7 sensitized target cells for the lysis by the CTL at concentrations between 0.03-30 µM (Fig. 2C). The lysis was approaching a plateau in the presence of C7 at concentrations above 1 µM. Recognition of the same 16-residue peptide by CD8⁺ T cell with class I MHC molecules restriction could represent presentation of a portion of the peptide by MHC molecules (20,25,55). To begin to map the peptide more finely, based on the observation that class I MHC molecules, including D^{d} in this case, tend to present peptides of 8-10 residues in length (15,22,26), we synthesized all seven overlapping decapeptides within C7, overlapping by 9 residues each (see sequences in legend to Fig. 2C) as well as a nonapeptide C7A2 corresponding to the HLA-A2 motif (See Fig. 1 and results below). Of these, only C7-A10 (LMGYIPLVGA) was more active than the full-length C7 peptide (Fig. 2C). Because neither of the overlapping decapeptides C7-G10 and C7-P10M is as active, we conclude that neither nonapeptide contained in these overlaps is sufficient for optimal response. Therefore, the decapeptide C7-A10 appears to be the optimal peptide for recognition by these D^{d}-restricted CTL. Interestingly, this peptide does not contain the D^{d} motif as defined by endogenous peptides eluted from D^{d} (15) (see below).

We have induced murine CTL with ability to kill syngeneic target cells expressing the HCV core protein as well as targets incubated in the presence of peptide C7 (HCV residues 129-144 within core), in H-2^{d} mice, but not in H-2^{b} mice. We conclude that H-2^{d} is an immune response (Ir) gene responder haplotype to C7 whereas H-2^{b} is not a responder. To determine which of the three H-2^{d} class I molecules presents C7, we used L cell (H-2^{k}) transfectants expressing K^{d}, L^{d}, or D^{d}, and found that the C7 peptide required the D^{d} molecule for effective peptide presentation. Interestingly, the same MHC molecule was found to present C7 (or more specifically C7-A10) here and the peptides P17 from HCV NS5 (60), as well as P18 (63) and HP53 from HIV-1 gp160 (61), which share no striking similarity in sequences except similarity in amphipathicity profiles when folded as an alpha helix. Of these four peptides, only P18 has a clear D^{d} binding motif as defined by endogenous peptides eluted from D^{d}, XGPX[K/R/H]XXX(X)[L/I/F], SEQ. ID. NO.: 9 (15). Thus, other motifs for binding D^{d} must exist as well. The identification of such novel D^{d}-binding peptides will aid in the characterization of such new motifs. Although insufficient homology is present to define an obvious motif for D^{d} binding, analysis of residues involved in D^{d} binding (63) for each peptide may shed new light on the structural requirements for the D^{d} specificity.

To get the maximal killing, the peptide concentration required to stimulate CTL in vitro secondarily or to sensitize targets appeared to be 1 - 30 µM for both C7 and C7-A10. This result suggests that these peptides bind with only moderate affinity to H-2D^{d}, compared with P18, an immunodominant CTL determinant of HIV-1 gp160 restricted by D^{d} (62) with the D^{d} motif. Modifications of C7 or C7-A10 may be found which more closely approximate the D^{d} motif that might bind with higher affinity and stimulate more efficiently (5-7). Also, C7 was not directly toxic to the cells in the absence of CTL.

### Example 2: Identification Of Human Patients Exposed To HCV

Having identified an epitope for murine CTL, we wanted to know whether it would be recognized by T cells from HCV seropositive patients as well. PBL from 8 HCV-seropositive patients, 2 individuals with chronic hepatitis B, and 2 healthy individuals were tested, with stimulation *in vitro,* for the lysis of autologous EBV-transformed cells or Con A blasts incubated with peptides at 10 µM (Table 4).

**Human CTL**. We selected 8 individuals, patients from the Kagawa Medical School Medical Center (Kagawa, Japan), with HCV-specific serum antibodies detected by anti-C100-3 (HCV Ab test, Ortho Diagnostic Systems) or second-generation enzyme immunoassay (EIA) tests (Abbott Laboratories, North Chicago, IL) specific for the putative core, NS3, and NS4 HCV proteins (C22,C33,and C100-3 antigens) and serum HCV RNA detected by the double polymerase chain reaction method with two pairs of external and internal (nested) primers deduced from the 5'-non-coding region (49). Individuals coinfected with hepatotrophic viruses other than HCV detected by serological testing were excluded from the study. We tested seven patients with chronic hepatitis C who had elevated serum levels of alanine aminotransferase (ALT) (80 to 450 IU/L for >1 year and one patient (#8) with acute hepatitis C who had recent onset of acute hepatitis with high serum level of ALT (1054 IU/L) and was PCR-positive for HCV and seropositive by second generation EIA, but had no prior clinical history of hepatitis (Table 4). Two patients with chronic hepatitis B (non C) detected by radioimmunoassay tests (positive for HBsAg and HBeAg; Abbott Laboratories, North Chicago, IL) and two healthy individuals seronegative for HCV and HBsAg were also tested.

**TABLE 4.**

| The response of PBL from HCV-seropositive individuals to C7 | | | | | | | | | | | | | | | |
|---|---|---|---|---|---|---|---|---|---|---|---|---|---|---|---|
| Patient | Age | Sex | ALT | anti-C100-3 | % specific lysis | | | | | | | | | | |
| no | (yr) | | (IU/L) | (unit) | C1 | C2 | C3 | C4 | C5 | C6 | C7 | C8 | C9 | C10 | C11 |
| 1 | 54 | M | 358 | 35.0 | | | | | | | 2 | | | | |
| 2 | 49 | M | 119 | 16.0 | | | | | | | -2 | | | | |
| 3 | 44 | M | 132 | 18.0 | 2 | 2 | 2 | 2 | 2 | 2 | 0 | 3 | 4 | 3 | 3 |
| 4 | 66 | M | 80 | 12.0 | 5 | 2 | 2 | 1 | 1 | 2 | 3 | | | | |
| 5 | 32 | F | 106 | 17.0 | 1 | 3 | 4 | 4 | 4 | 3 | 2 | 4 | 4 | 3 | 5 |
| 6 | 21 | F | 450 | 24.0 | 1 | 2 | 1 | 0 | -1 | 1 | 0 | 1 | 2 | 1 | 0 |
| 7¶ | 40 | M | 257 | 25.0 | 3 | 3 | 3 | 4 | 4 | 4 | 20 | 2 | 2 | 3 | 2 |
| | | | | | | | | | | | #42 | | | | |
| 8¶ | 58 | F | 1054 | 0.0* | 4 | 4 | 4 | 4 | 5 | 4 | 25 | 4 | 4 | 4 | 4 |
| 9§ | 43 | M | 225 | | | | | | | | 2 | | | | |
| 10§ | 49 | M | 249 | | | | | | | | -2 | | | | |
| 11† | 28 | F | 17 | | 0 | -1 | 0 | 0 | 1 | -2 | 2 | 1 | 1 | 1 | 0 |
| 12† | 30 | M | 22 | | | | | | | | 4 | | | | |

| | | | | | | | | | | | | | | | |
|---|---|---|---|---|---|---|---|---|---|---|---|---|---|---|---|
| PBL were stimulated in vitro with mitomycin C-treated PBL and each peptide 10µM, and tested against autologous target cells in the presence or absence of the corresponding peptide (10µM) at an E/T ratio of 100:1, as described in "Material and Methods". No toxicity of peptide against targets was observed. Two patients with chronic hepatitis B§ and two healthy individuals† did not show any response to C7 (<4%), ConA blast targets from FBL were used in patients No. 2, 5, and 8. Data are the means of triplicate samples with an SE of less than 5% and are representative of at least two independent experiments. *HCV RNA⁺ and positive by second generation ElA, # PBL were stimulated twice in vitro. , | | | | | | | | | | | | | | | |
| ¶HLA-A2.1 positive | | | | | | | | | | | | | | | |

In the human assays, lines derived from peripheral blood lymphocytes (PBL) of donors by Epstein-Barr virus (EBV) transformation (12) or Con A blast targets made from autologous PBL, as described previously (61), were used as targets either in the presence of a peptide (10 µM) or after infection with vHCV#4 (61). The PBL were separated on lymphocyte separating medium (LeucoPREP, Becton Dickinson, Mountain View, CA). The PBL (4x10⁶/ml in 24-well culture plates) were stimulated with mitomycin C-treated PBL of the same donor (2x10⁶/ml in 24-well culture plates) in the presence of 10 µM peptide at day 1 and 8, and 50 units/ml of human rIL2 (Cetus Corp., Emeryville, CA) added at day 2, 9, and 12 with fresh CTM. At day 8 or 15 of the culture, the stimulated PBL were used as effectors and tested on targets of the EBV transformed lines or Con A blasts labeled overnight with 0.1 mCi of ⁵¹Cr. The 6-h ⁵¹Cr release assay was performed as described above.

The PBL from two patients (#7 and #8) were able to specifically kill targets in the presence of C7 but not in the presence of other peptides or medium alone. None of the peptides tested was recognized by four other patients. Conversely, the PBL from four HCV-seronegative donors failed to kill the targets with the C7 peptide (Table 4). The patients with acute and chronic hepatitis also showed cytotoxic activity against the targets infected with vHCV#4 and endogenously expressing HCV core protein.

For one of these two donors we were able to test the phenotype of the effector cells. CTL activity from responder patient #7 with chronic hepatitis was blocked by anti-HLA class I (W6/32, IgG2a) but not by anti-HLA class II DR (L-243, IgG2a) (Fig.3). Thus, it is expected that these are conventional antigen-specific HLA class I-restricted CD8⁺ CTLs. Patients #7 and #8 were HLA typed and found to express HLA-A1, A2, B51, Bw4, Bw6 and HLA-A2, B51, respectively. Moreover, we noted that the C7 peptide sequence contained an HLA-A2-binding motif (22,34), with the sequence DLMGYIPLV. To ask whether the recognition of C7 by these patients' CTL was due to presentation of this nonamer sequence by HLA-A2, we synthesized the corresponding nonamer peptide, designated C7A2, and tested recognition of both this and the full-length 16-mer, C7, by Patient #7 and #8 CTL on targets sharing or not sharing HLA-A2 (Fig. 4). The CTL killed autologous targets in the presence of C7 or the nonamer C7A2, and also the HLA-A-and-B-negative cell line C1R transfected with HLA-A2, but not untransfected C1R or allogeneic targets lacking HLA-A2 (Fig. 4). Therefore, we conclude that the C7 peptide contains an HLA-A2 motif-positive nonamer which is presented by HLA-A2 to human CTL of HCV-infected acute and chronic hepatitis patients.

These data showed that this peptide, recognized in the context of one murine MHC haplotype, can also be recognized in association with a human MHC molecule. Recognition of the same 16-residue peptide by different T cells with both mouse and human class I MHC molecules could represent presentation of the same broadly recognized site by multiple class I molecules or could represent presentation of different partially overlapping positions of the peptide by different MHC molecules (20,25,55).

To distinguish these possibilities, we performed fine mapping of the murine C7 epitope using seven overlapping decapeptides spanning the 16-residue peptide (see Fig. 2c). The only peptide that was as active or more active than the 16-mer was C7-A10. Indeed, this decapeptide appears to be the minimal peptide for the murine CTL, because the two nonapeptides contained within C7-A10 are also contained in one or the other of the overlapping peptides C7-G10 and C7-P10M, which were much less active. (It is unlikely that these other decapeptides are inactive because of a failure in processing, since the whole 16-mer is processed to an active form under the same conditions.) In comparison, in the case of the human response restricted by HLA-A2, we have identified at least one nonamer epitope as the peptide DLMGYIPLV, which contains an HLA-A2-binding motif with anchor residues at positions 2 and 9 (22,34). This peptide constitutes the minimal human HLA-A2-restricted CTL epitope in this portion of the HCV core protein. However, because the H-2^{d} mice do not respond to this nonamer peptide (Fig. 2c) and respond only weakly to the C7-G10 and C7-V10 peptides that contain this nonamer (Fig. 2c), the minimal epitopes recognized by mice and humans must not be identical, but closely overlapping. Indeed, all but one residue of the human epitope are contained within the murine epitope. The similarity of core residues of C7 for recognition by mice and human is striking.

Recent reports of HCV sequence diversity allow comparison of several isolates ((11,29,42,48,70), reviewed in (33)). The core protein is well conserved relative to the highly variable envelope glycoproteins E1 and E2. This hypervariability of the HCV envelope proteins suggests that these surface proteins may be under immunologic selective pressure for variation, as has been suggested in the case of the HIV-1 envelope protein V3 loop, which is the principal neutralizing antibody domain as well as a determinant for CTL in both the human and the mouse (12,27,50,57,62). However, within the groups of HCV isolates (broadly subdivided by comparison of all the reported HCV sequences) the core shows 95% to 100% sequence identity (33).

There are many lines of evidence that CTL can block outgrowth of virus (19,35,44,47,51,52,68,69). Because it is so well conserved, peptides from the HCV core protein, presented by class I MHC molecules to CD8⁺CD4⁻ CTL of both mice and humans, are likely to be useful as a component of a broadly effective vaccine for HCV, especially because HLA-A2 is the most prevalent human class I molecule, present in about 46% of the United States population. Also, in a small preliminary sampling of Japanese patients with hepatitis C in one of our labs, 15 of 23 were HLA-A2⁺.

The prevalence of the HLA-A2 haplotype in human populations, together with the strong conservation of the C7 peptide among HCV isolates, indicates that the peptide C7 is useful as a diagnostic reagent in a large proportion of the human population to detect exposure or infection with HCV of many strains.

It will be important to assess infected patients for a possible correlation between the clinical course of hepatitis and the response to this peptide in HLA-A2-positive, HCV infected patients. Such analysis may reveal information of prognostic value. The presence of C7-specific CTL in a patient infected with HCV, might provide information as to whether the patient will clear the virus rapidly or go on to develop chronic liver disease. This information could be useful in planning further treatment.

In our previous experience with HIV-1 proteins gp160 and reverse transcriptase, the epitopes seen by murine CTL were also seen by human CTL (12,30,62). This is relevant in that this finding implies that the present method for identifying CTL epitopes in mice is generally useful for identification of peptides that are useful as vaccines and diagnostic reagents for human HCV retroviral infections.

### Example 3: Additional Diagnostic Methods Using The Peptides Of The Present Invention

In addition to measurement of specific lysis of target cells by CTL specific for the peptide reagent, other activities of CTL associated with antigen recognition can be assayed. For example, cytokines secreted in response to antigen activation of CTL can be assayed. A particularly preferred cytokine to be measured is γ-interferon. Methods for measuring particular cytokines are well-known in the art. Two preferred formats are immunoassay of the cytokine and measurement of proliferation of a cytokine dependent cell line.

Furthermore, the method described in Example 2 can be modified by elimination of the step of transforming the PBL from the patient with EBV. The viral transformation is done for laboratory purposes to establish reproducible cell lines. For clinical assay purposes, the PBL can be cultured in the short term to sufficient numbers without transformation. Autologous concanavalin A-stimulated PBL blasts can be used as targets instead of EBV-transformed B-lymphoblastoid cells. Also, targets are unnecessary in assays measuring γ-interferon secretion as the response of the antigen-specific CTL.

### Example 4: Formulation Of Peptides As Pharmaceutical Compositions

The peptides of the present invention can be admixed with any pharmaceutically acceptable carrier, adjuvant or diluent. Preferably, pharmaceutical compositions of the present invention are prepared for intravenous, subcutaneous, intramuscular or intradermal injection. In such formulations, the peptides are solubilized at such concentration as provides a dose ranging from 50 to 500 µg. The solvent can be sterile saline or any other pharmaceutically acceptable solvent.

As to adjuvants, Incomplete Freund's Adjuvant (for subcutaneous, intramuscular or intradermal injection) and QS21 can be used with both peptide and the whole protein. Alum can also be used as an adjuvant with the whole protein priming immunizations.

The peptides can be modified by coupling to a lipid tail, as described by Deres, K. et al., Nature 342:561-564 (1989).

Furthermore, for intravenous injection, the peptides might be modified to provide resistance to proteolytic degradation. A typical modification is to amidate the carboxyl terminus of the peptide. Methods for accomplishing these derivatizations of peptides are well-known in the art.

### Example 5: Administration Of Peptides As Vaccines For Prevention Of HCV Infection

The core protein and peptides, formulated as described in Example 4, are administered as part of typical vaccination protocols. Subjects are first primed by administration of HCV core protein, then boosted with administrations of the peptide. Whole protein administration is performed by injection, preferably intramuscularly or subcutaneously. In addition to direct injection, peptides can also be administered by incubating the peptide with autologous PBMC for 2 hours, irradiating the incubated cells, and reinfusing intravenously, as described in Takehashi et al., International Immunology, 5:849-857, 1993 and in WO-A-94 21287.

The HCV core protein can be provided by a recombinant vector expressing the entire protein. The recombinant vector is not particularly limited, but a preferred embodiment of the vector is one in which DNA encoding the HCV core protein is expressed from a vaccinia virus vector. The vHCV#4 vector, described in Example 1, is particularly preferred. The recombinant vector is administered by injection, either intravenously or intradermally. Alternatively, cells of the patient can be transformed with the vector and the transformed cells can be infused into the patient or implanted under the skin.

Some time following priming, preferably one to two weeks later, the patient is then immunized with a peptide according to the present invention. The peptide is administered in a sufficient amount to elicit a CTL response to the immunizing peptide, as determined by any known method for assaying such a CTL response. The method described in Example 2 is entirely suitable. Typically the peptide is administered in an amount of 50 to 500 µg. Repeated boosting can be performed.

### REFERENCES

The following articles of the scientific literature are cited in the present Specification. Each of these articles is hereby incorporated in its entirety by such reference.
1. **Abastado, J. -P., C. Jaulin, M. -P. Schutze, P. Langlade-Demoyen, F. Plata, K. Ozato, and P. Kourilsky**, J. Exp. Med.166:327-340 (1987).
2. **Alter, H. J., R. H. Purcell, J. W. Shih, J. C. Melpolder, M. Houghton, Q. -L. Choo, and G. Kuo.**, N. Engl. J. Med. **321**:1494-1500 (1989).
3. **Benacerraf, B.**, J. Immunol. **120**:1809-1812 (1978).
4. **Berzofsky, J. A.**, J. Acq. Immune Defic. Syndromes **4**:451-459 (1991).
5. **Berzofsky, J. A.**, Annals N. Y. Acad. Sci. **690**:256-264 (1993).
6. **Bodmer, H. C., R. M. Pemberton, J. B. Rothbard, and B. A. Askonas.**, Cell **52**:253-258 (1988).
7. **Boehncke, W. -H., T. Takeshita, C. D. Pendleton, S. Sadegh-Nasseri, L. Racioppi, R. A. Houghten, J. A. Berzofsky, and R. N. Germain.**, J. Immunol. **150**:331-341 (1993).
8. **Bradley, D. W., K. A. McCaustland, E. H. Cook, C. A. Schable, J. W. Ebert, and J. E. Maynard.**, Gastroenterology **88**:773-779 (1985).
9. **Chakrabarti, S., M. Robert-Guroff, F. Wong-Staal, R. C. Gallo, and B. Moss**, Nature **320**:535-537 (1986).
10. **Choo, Q. -L., G. Kuo, A. J. Weiner, L. R. Overby, D. W. Bradley, and M. Houghton.**, Science **244**:359-362 (1989).
11. **Christiano, K., A. M. Di Bisceglie, J. H. Hoofnagle, and S. M. Feinstone.**, Hepatology **14**:51-55 (1991).
12. **Clerici, M., D. R. Lucey, R. A. Zajac, R. N. Boswell, H. M. Gebel, H. Takahashi, J. Berzofsky, and G. M. Shearer**., J. Immunol. **146**:2214-2219 (1991).
13. **Cornette, J. L., H. Margalit, C. DeLisi, and J. A. Berzofsky**., Methods in Enzymol. **178**:611-634 (1989).
14. **Cornette, J. L., H. Margalit, C. DeLisi, and J. A. Berzofsky.**, The amphipathic Helix as a structural feature involved in T-cell recognition, p. 333-346. In R. M. Epand (ed.), The Amphipathic Helix. CRC Press, Boca Raton (1993).
15. **Corr, M., L. F. Boyd, E. A. Padlan, and D. H. Margulies**, J. Exp. Med. **178**:1877-1892 (1993).
16. **DeGroot, A. S., M. Clerici, A. Hosmalin, S. H. Hughes, D. Barnd, C. W. Hendrix, R. A. Houghten, G. M. Shearer, and J. A. Berzofsky.**, J. Infect. Dis. **164**:1058-1065 (1991).
17. **DeLisi, C. and J. A. Berzofsky**, Proc. Natl. Acad. Sci. U. S. A. **82**:7048-7052 (1985).
18. **Di Bisceglie, A. M. and J. H. Hoofnagle**., Hepatology **13**:601-603 (1991).
19. **Earl, P. L., B. Moss, R. P. Morrison, K. Wehrly, J. Nishio, and B. Chesebro**., Science **234**:728 (1986).
20. **Elliott, T., V. Cerundolo, J. Elivn, and A. Townsend**, Nature **351**:402-406 (1991).
21. **Evans, G. A., D. H. Margulies, B. Shykind, J. G. Seidman, and K. Ozato**, Nature **300**:755-757 (1982).
22. **Falk, K., O. Rötzschke, S. Stevanovic, G. Jung, and H. -G. Rammensee**, Nature **351**:290-296 (1991).
23. **Farci, P., H. J. Alter, S. Govindarajan, D. C. Wong, R. Engle, R. R. Lesniewski, I. K. Mushahwar, S. M. Desai, R. H. Miller, N. Ogata, and R. H. Purcell**, Science **258**:135-140 (1992).
24. **Feinstone, S. M., H. J. Alter, H. P. Dienes, Y. Shimizu, H. Popper, D. Blackmore, D. Sly, W. T. London, and R. H. Purcell**, J. Infect. Dis. **144**:588-598 (1981).
25. **Gammon, G., H. M. Geysen, R. J. Apple, E. Pickett, M. Palmer, A. Ametani, and E. E. Sercarz**, J. Exp. Med. **173**:609-617 (1991).
26. **Germain, R. N. and D. H. Margulies**, Annu. Rev. Immunol. **11**:403-450 (1993).
27. **Goudsmit, J., C. Debouck, R. H. Meloen, L. Smit, M. Bakker, D. M. Asher, A. V. Wolff, C. J. Gibbs, Jr., and D. C. Gajdusek**, Proc. Natl. Acad. Sci. U. S. A. **85**:4478-4482 (1988).
28. **He, L. -F., D. Alling, T. Popkin, M. Shapiro, H. J. Alter, and R. H. Purcell**, J. Infect. Dis. **156**:636-640 (1987).
29. **Hijikata, M., N. Kato, Y. Ootsuyama, M. Nakagawa, S. Ohkoshi, and K. Shimotohno**, Biochem. Biophys. Res. Commun. **175**:220-228 (1991).
30. **Hosmalin, A., M. Clerici, R. Houghten, C. D. Pendleton, C. Flexner, D. R. Lucey, B. Moss, R. N. Germain, G. M. Shearer, and J. A. Berzofsky**, Proc. Natl. Acad. Sci. U. S. A. **87**:2344-2348 (1990).
31. **Houghten, R. A.**, Proc. Natl. Acad. Sci. USA. **82**:5131-5135 (1985).
32. **Houghton, M., Q. -L. Choo, and G. Kuo**, European Patent Application **88310922.5**:31-8216 (1988).
33. **Houghton, M., A. Weiner, J. Han, G. Kuo, and Q. -L. Choo**, Hepatology **14**:381-388 (1991).
34. **Hunt, D. F., R. A. Henderson, J. Shabanowitz, K. Sakaguchi, H. Michel, N. Sevilir, A. L. Cox, E. Appella, and V. H. Engelhard**, Science **255**:1261-1263 (1992).
35. **Imawari, M., M. Nomura, T. Kaieda, T. Moriyama, K. Oshimi, I. Nakamura, T. Gunji, S. Ohnishi, T. Ishikawa, H. Nakagama, and F. Takaku**, Proc. Natl. Acad. Sci. USA **86**:2883-2887 (1989).
36. **Kato, N., M. Hijikata, Y. Ootsuyama, M. Nakagawa, S. Ohkoshi, T. Sugimura, and K. Shimotohno**, Proc. Natl Acad. Sci. USA **87**:9524-9528 (1990).
37. **Kiyosawa, K., T. Sodeyama, E. Tanaka, Y. Gibo, K. Yoshizawa, Y. Nakano, S. Furuta, Y. Akahane, K. Nishioka, R. H. Purcell, and H. J. Alter**, Hepatology **12**:671-675 (1990).
38. **Koziel, M. J., D. Dudley, J. T. Wong, J. Dienstag, M. Houghton, R. Ralston, and B. D. Walker**, J. Immunol. **149**:3339-3344 (1992).
39. **Le, A. -X. T., E. J. Bernhard, M. J. Holterman, S. Strub, P. Parham, E. Lacy, and V. H. Engelhard**, J. Immunol. **142**:1366-1371 (1989).
40. **Margalit, H., J. L. Spouge, J. L. Cornette, K. Cease, C. DeLisi, and J. A. Berzofsky**, J. Immunol. **138**:2213-2229 (1987).
41. **Margulies, D. H., G. A. Evans, K. Ozato, R. D. Camerini-Otero, K. Tanaka, E. Appella, and J. G. Seidman**, J. Immunol. **130**:463 (1983).
42. **Maéno, M., K. Kaminaka, H. Sugimoto, M. Esumi, N. Hayashi, K. Komatsu, K. Abe, S. Sekiguchi, M. Yano, K. Mizuno, and T. Shikata**, Nucleic Acids Research **18**:2685-2689 (1990).
43. **McCluskey, J., L. Boyd, M. Foo, J. Forman, D. H. Margulies, and J. A. Bluestone**, J. Immunol. **137**:3881-3890 (1986).
44. **Mondelli, M., G. M. Vergani, A. Alberti, D. Vergani, B. Portmann, A. L. W. F. Eddleston, and R. Williams**, J. Immunol. **129**:2773-2778 (1982).
45. **Murre, C., E. Choi, J. Weis, J. G. Seidman, K. Ozato, L. Liu, S. J. Burakoff, and C. S. Reiss**, J. Exp. Med. **160**:167-178 (1984).
46. **Myers, G., S. F. Josephs, J. A. Berzofsky, A. B. Rabson, T. F. Smith, and F. Wong-Staal**, Human retroviruses and AIDS 1989. Los Alamos National Laboratory, New Mexico (1989).
47. **Naumov, N. V., M. Mondelli, G. J. M. Alexander, R. S. Tedder, A. L. W. F. Eddleston, and R. Williams**, Hepatology **4**:63-68 (1984).
48. **Ogata, N., H. J. Alter, R. H. Miller, and R. H. Purcell**, Proc. Natl. Acad. Sci. USA **88**:3392-3396 (1991).
49. **Okamoto, H., S. Okada, Y. Sugiyama, T. Tanaka, Y. Sugai, Y. Akahane, A. Machida, S. Mishiro, H. Yoshizawa, Y. Miyakawa, and M. Nayumi**, Japanese J. Exp. Med. **60**:215-222 (1990).
50. **Palker, T. J., M. E. Clark, A. J. Langlois, T. J. Matthews, K. J. Weinhold, R. R. Randall, D. P. Bolognesi, and B. F. Haynes**, Proc. Natl. Acad. Sci. U. S. A. **85**:1932-1936 (1988).
51. **Pasternack, M. S**., Adv. Intern. Med. **33**:17-44 (1988).
52. **Plata, F., P. Langlade-Demoyen, J. P. Abastado, T. Berbar, and P. Kourilsky**, Cell **48**:231-240 (1987).
53. **Prince, A. M., B. Brotman, T. Huima, D. Pascual, M. Jaffery, and G. Inchauspé**, J. Infect. Dis. **165**:438-443 (1992).
54. **Realdi, G., A. Alberti, M. Rugge, A. M. Rigoli, F. Tremolada, L. Schivazappa, and A. Ruol**, Gut **23**:270-275 (1982).
55. **Reddehase, M. J., J. B. Rothbard, and U. H. Koszinowski**, Nature **337**:651-653 (1989).
56. **Rosenthal, A. S**., Immunol. Rev. **40**:136-152 (1978).
57. **Rusche, J. R., K. Javaherian, C. McDanal, J. Petro, D. L. Lynn, R. Grimaila, A. Langlois, R. C. Gallo, L. O. Arthur, P. J. Fischinger, D. P. Bolognesi, S. D. Putney, and T. J. Matthews**, Proc. Natl. Acad. Sci. U. S. A. **85**:3198-3202 (1988).
58. **Sarmiento, M., A. L. Glasebrook, and F. W. Fitch**, J. Immunol. **125**:2665-2672 (1980).
59. **Schwartz, R. H.**, Annu. Rev. Immunol. **3**:237-261 (1985).
60. **Shirai, M., T. Akatsuka, C. D. Pendleton, R. Houghten, C. Wychowski, K. Mihalik, S. Feinstone, and J. A. Berzofsky** , J. Virol. **66**:4098-4106 (1992).
61. **Shirai, M., C. D. Pendleton, and J. A. Berzofsky**, J. Immunol. **148**:1657-1667 (1992).
62. **Takahashi, H., J. Cohen, A. Hosmalin, K. B. Cease, R. Houghten, J. Cornette, C. DeLisi, B. Moss, R. N. Germain, and J. A. Berzofsky**, Proc. Natl. Acad. Sci. USA **85**:3105-3109 (1988).
63. **Takahashi, H., R. Houghten, S. D. Putney, D. H. Margulies, B. Moss, R. N. Germain, and J. A. Berzofsky**, J. Exp. Med. **170**:2023-2035 (1989).
64. **Takahashi, H., S. Merli, S. D. Putney, R. Houghten, B. Moss, R. N. Germain, and J. A. Berzofsky**, Science **246**:118-121 (1989).
65. **Takamizawa, A., C. Mori, I. Fuke, S. Manabe, S. Murakami, J. Fujita, E. Onishi, T. Andoh, I. Yoshida, and H. Okayama**, J. Virol. **65**:1105-1113 (1991).
66. **Townsend, A. and H. Bodmer**, Annu. Rev. Immunol. **7**:601-624 (1989).
67. **Townsend, A. RM., J. Rothbard, F. M. Gotch, G. Bahadur, D. Wraith, and A. J. McMichael**, Cell **44**:959-968 (1986).
68. **Tsubota, H., C. I. Lord, D. I. Watkins, C. Morimoto, and N. L. Letvin**, J. Exp. Med. **169**:1421-1434 (1989).
69. **Walker, C. M., D. J. Moody, D. P. Stites, and J. A. Levy**, Science **234**:1563-1566 (1986).
70. **Weiner, A. J., M. J. Brauer, J. Rosenblatt, K. H. Richman, J. Tung, K. Crawford, F. Bonino, G. Saracco, Q. -L. Choo, M. Houghton, and J. H. Han**, Virology **180**:842-848 (1991).
71. **Weiner, A. J., H. M. Geysen, C. Christopherson, J. E. Hall, T. J. Mason, G. Saracco, F. Bonino, K. Crawford, C. D. Marion, K. A. Crawford, M. Brunetto, P. J. Barr, T. Miyamura, J. McHutchinson, and M. Houghton**, Proc. Natl. Acad. Sci. U. S. A. **89**:3468-3472 (1992).
72. **Wilde, D. B., P. Marrack, J. Kappler, D. P. Dialynas, and F. W. Fitch**, J. Immunol. **131**:2178-2183 (1983).
73. **Zinkernagel, R. M. and P. C. Doherty**, Adv. Immunol. **27**:51-177 (1979).
74. **Takehashi, H. et al**., International Immunology, **5**:849-857 (1993).
75. **Margalit, H. et al.**, J. Immunol. **138**:2213-2229 (1987).

## Claims

1. A composition comprising a purified peptide having an amino acid sequence selected from the group consisting of SEQ. I.D. NO. 4 and SEQ. I.D. NO. 17, and a carrier, wherein said purified peptide is present in an amount effective for stimulating a cytotoxic T lymphocyte response in a mammal.

2. The composition of claim 1, wherein said carrier is the adjuvant QS21.

3. The composition of claim 1, wherein said carrier comprises irradiated autologous peripheral blood mononuclear cells.

4. A composition comprising a purified peptide having an amino acid sequence of SEQ ID NO. 10 and the adjuvant QS21, wherein said purified peptide is present in an amount effective for stimulating a cytotoxic T lymphocyte response in a mammal.

5. The composition of any one of claims 1 to 4, wherein said peptide is present in an amount providing a dose ranging from 50 to 500 µg.

6. The composition of any one of claims 1 to 5, wherein said mammal is a human.

7. A pharmaceutical composition comprising a peptide according to Claim 1 and a pharmaceutically acceptable carrier.

8. A method for diagnosing exposure of a patient to Hepatitis C Virus or for predicting a patient's clinical course following Hepatitis C Virus infection, which comprises:
i) obtaining a sample of peripheral blood mononuclear cells from said patient;
ii) culturing said peripheral blood mononuclear cells together with a peptide according to claim 1 under conditions which promote the proliferation of cytotoxic T lymphocytes; and
iii) assaying the peptide specific cytotoxic T lymphocyte activity in said culture.

9. The method according to claim 8, wherein said assay for peptide specific cytotoxic T lymphocyte activity is selected from the group of assaying target cell lysis, assaying secretion of a cytokine, and assaying proliferation of cytotoxic T lymphocytes.

10. Use of a peptide having the amino acid sequence of SEQ. I.D. NO. 10 for the preparation of a medicament to induce a cytotoxic T lymphocyte response.

11. Use of a peptide having the amino acid sequence of SEQ. I.D. NO. 4 for the preparation of a medicament to induce a cytotoxic T lymphocyte response.

12. Use of a peptide having the amino acid sequence of SEQ. I.D. NO. 17 for the preparation of a medicament to induce a cytotoxic T lymphocyte response.

13. A vaccine composition capable of inducing a cytotoxic T lymphocyte response, comprising a peptide having the amino acid sequence of SEQ. I. D. NO. 10 and a pharmaceutically acceptable carrier.

14. A vaccine composition capable of inducing a cytotoxic T lymphocyte response, comprising a peptide having the amino acid sequence of SEQ. I. D. NO. 4 and a pharmaceutically acceptable carrier.

15. A vaccine composition capable of inducing a cytotoxic T lymphocyte response, comprising a peptide having the amino acid sequence of SEQ. I. D. NO. 17 and a pharmaceutically acceptable carrier.

16. A pharmaceutical composition, capable of inducing a cytotoxic T lymphocyte response, comprising a peptide having the amino acid sequence of SEQ. I. D. NO. 10 and a pharmaceutically acceptable carrier.

17. A pharmaceutical composition, capable of inducing a cytotoxic T lymphocyte response, comprising a peptide having the amino acid sequence of SEQ. I. D. NO. 4 and a pharmaceutically acceptable carrier.

18. A pharmaceutical composition, capable of inducing a cytotoxic T lymphocyte response, comprising a peptide having the amino acid sequence of SEQ. I. D. NO. 17 and a pharmaceutically acceptable carrier.

19. Use of a peptide having the amino acid sequence of SEQ. I.D. NO. 10 and a pharmaceutically acceptable carrier for the preparation of a vaccine composition capable of inducing a cytotoxic T lymphocyte response for use in preventing Hepatitis C Viral infections.

20. Use of a peptide having the amino acid sequence of SEQ. I.D. NO. 4 and a pharmaceutically acceptable carrier for the preparation of a vaccine composition capable of inducing a cytotoxic T lymphocyte response for use in preventing Hepatitis C Viral infections.

21. Use of a peptide having the amino acid sequence of SEQ. I.D. NO. 17 and a pharmaceutically acceptable carrier for the preparation of a vaccine composition capable of inducing a cytotoxic T lymphocyte response for use in preventing Hepatitis C Viral infections.

## Patentansprüche

1. Zusammensetzung, die ein gereinigtes Peptid mit einer Aminosäuresequenz, ausgewählt aus der Gruppe, bestehend aus SEQ. I.D. NO. 4 und SEQ. I.D. NO. 17, und einen Träger umfaßt, wobei das gereinigte Peptid in einer Menge, die wirksam ist, um eine zytotoxische Reaktion in einem Säugetier zu stimulieren, vorliegt.

2. Zusammensetzung nach Anspruch 1, wobei der Träger das Adjuvans QS21 ist.

3. Zusammensetzung nach Anspruch 1, wobei der Träger bestrahlte autologe mononukleäre Zellen des peripheren Bluts umfaßt.

4. Zusammensetzung, die ein gereinigtes Peptid mit einer Aminosäuresequenz von SEQ ID NO. 10 und das Adjuvans QS21 umfaßt, wobei das gereinigte Peptid in einer Menge, die wirksam ist, um eine zytotoxische Reaktion in einem Säugetier zu stimulieren, vorliegt.

5. Zusammensetzung nach einem der Ansprüche 1 bis 4, wobei das Peptid in einer Menge, die eine von 50 bis 500 µg reichende Dosis bereitstellt, vorliegt.

6. Zusammensetzung nach einem der Ansprüche 1 bis 5, wobei das Säugetier ein Mensch ist.

7. Pharmazeutische Zusammensetzung, die ein Peptid nach Anspruch 1 und einen pharmazeutisch annehmbaren Träger umfaßt.

8. Verfahren zum Diagnostizieren einer Exposition eines Patienten gegenüber dem Hepatitis C-Virus oder zum Vorhersagen des klinischen Verlaufs bei einem Patienten nach einer Infektion durch das Hepatitis C-Virus, welches umfaßt:
i) Gewinnen einer Probe von mononukleären Zellen des peripheren Bluts von dem Patienten;
ii) Kultivieren der mononukleären Zellen des peripheren Bluts zusammen mit einem Peptid nach Anspruch 1 unter Bedingungen, die die Proliferation von zytotoxischen T-Lymphozyten fördern; und
iii) Untersuchen der Peptid-spezifischen zytotoxischen T-Lymphozytenaktivität in der Kultur.

9. Verfahren nach Anspruch 8, wobei dem Dosierung auf Peptid-spezifische zytotoxische T-Lymphozytenaktivität ausgewählt wird aus der Gruppe einem Dosierung einer Zielzelllyse, einem Dosierung der Sekretion eines Zytokins und einem Dosierung der Proliferation von zytotoxischen T-Lymphozyten.

10. Verwendung eines Peptids mit der Aminosäuresequenz von SEQ. I.D. NO. 10 für die Herstellung eines Arzneimittels zur Induktion einer zytotoxischen Lymphozytenreaktion.

11. Verwendung eines Peptids mit der Aminosäuresequenz von SEQ. I.D. NO. 4 für die Herstellung eines Arzneimittels zur Induktion einer zytotoxischen Lymphozytenreaktion.

12. Verwendung eines Peptids mit der Aminosäuresequenz von SEQ. I.D. NO. 17 für die Herstellung eines Arzneimittels zur Induktion einer zytotoxischen Lymphozytenreaktion.

13. Impfstoffzusammensetzung, die in der Lage ist, eine zytotoxische T-Lymphozytenreaktion zu induzieren, welche ein Peptid mit der Aminosäuresequenz von SEQ. I.D. NO. 10 und einen pharmazeutisch annehmbaren Träger umfaßt.

14. Impfstoffzusammensetzung, die in der Lage ist, eine zytotoxische T-Lymphozytenreaktion zu induzieren, welche ein Peptid mit der Aminosäuresequenz von SEQ. I.D. NO. 4 und einen pharmazeutisch annehmbaren Träger umfaßt.

15. Impfstoffzusammensetzung, die in der Lage ist, eine zytotoxische T-Lymphozytenreaktion zu induzieren, welche ein Peptid mit der Aminosäuresequenz von SEQ. I.D. NO. 17 und einen pharmazeutisch annehmbaren Träger umfaßt.

16. Pharmazeutische Zusammensetzung, die in der Lage ist, eine zytotoxische T-Lymphozytenreaktion zu induzieren, welche ein Peptid mit der Aminosäuresequenz von SEQ. I.D. NO. 10 und einen pharmazeutisch annehmbaren Träger umfaßt.

17. Pharmazeutische Zusammensetzung, die in der Lage ist, eine zytotoxische T-Lymphozytenreaktion zu induzieren, welche ein Peptid mit der Aminosäuresequenz von SEQ. I.D. NO. 4 und einen pharmazeutisch annehmbaren Träger umfaßt.

18. Pharmazeutische Zusammensetzung, die in der Lage ist, eine zytotoxische T-Lymphozytenreaktion zu induzieren, welche ein Peptid mit der Aminosäuresequenz von SEQ. I.D. NO. 17 und einen pharmazeutisch annehmbaren Träger umfaßt.

19. Verwendung eines Peptids mit der Aminosäuresequenz von SEQ. I.D. NO. 10 und eines pharmazeutisch annehmbaren Trägers für die Herstellung einer Impfstoffzusammensetzung, die in der Lage ist, eine zytotoxische T-Lymphozytenreaktion zu induzieren, für eine Verwendung zum Verhindern von Infektionen durch das Hepatitis C-Virus.

20. Verwendung eines Peptids mit der Aminosäuresequenz von SEQ. I.D. NO. 4 und eines pharmazeutisch annehmbaren Trägers für die Herstellung einer Impfstoffzusammensetzung, die in der Lage ist, eine zytotoxische T-Lymphozytenreaktion zu induzieren, für eine Verwendung zum Verhindern von Infektionen durch das Hepatitis C-Virus.

21. Verwendung eines Peptids mit der Aminosäuresequenz von SEQ. I.D. NO. 17 und eines pharmazeutisch annehmbaren Trägers für die Herstellung einer Impfstoffzusammensetzung, die in der Lage ist, eine zytotoxische T-Lymphozytenreaktion zu induzieren, für eine Verwendung zum Verhindern von Infektionen durch das Hepatitis C-Virus.

## Revendications

1. Composition comprenant un peptide purifié ayant une séquence d'acides aminés choisie dans le groupe constitué de SEQ. I.D. n° 4 et SEQ. I.D. n° 17 et un véhicule, dans laquelle ledit peptide purifié est présent en une quantité efficace pour stimuler une réponse cytotoxique chez un mammifère.

2. Composition selon la revendication 1, dans laquelle ledit véhicule est l'adjuvant QS21.

3. Composition selon la revendication 1, dans laquelle ledit véhicule comprend des cellules mononucléaires de sang périphérique autologues irradiées.

4. Composition comprenant un peptide purifié ayant une séquence d'acides aminés de SEQ. ID n° 10 et l'adjuvant QS21, dans laquelle ledit peptide purifié est présent en une quantité efficace pour stimuler une réponse cytotoxique chez un mammifère.

5. Composition selon l'une quelconque des revendications 1 à 4, dans laquelle ledit peptide est présent en une quantité fournissant une dose dans la gamme de 50 à 500 µg.

6. Composition selon l'une quelconque des revendications 1 à 5, dans laquelle ledit mammifère est un Homme.

7. Composition pharmaceutique comprenant un peptide selon la revendication 1 et un véhicule acceptable sur le plan pharmaceutique.

8. Procédé pour diagnostiquer une exposition d'un patient au virus de l'hépatite C ou pour prévoir une évolution clinique de patient à la suite d'une infection par le virus de l'hépatite C, qui comprend les étapes comprenant :
i) l'obtention d'un échantillon de cellules mononucléaires de sang périphérique provenant dudit patient ;
ii) la culture desdites cellules mononucléaires de sang périphérique conjointement avec un peptide selon la revendication 1 dans des conditions qui favorisent la prolifération des lymphocytes T cytotoxiques ; et
iii) le dosage de l'activité des lymphocytes T cyto-toxiques spécifiques du peptide dans ladite culture.

9. Procédé selon la revendication 8, dans lequel ledit dosage concernant l'activité des lymphocytes T cytotoxiques spécifiques du peptide est choisi dans le groupe du dosage de lyse de cellules cibles, du dosage de sécrétion d'une cytokine et du dosage de la prolifération des lymphocytes T cytotoxiques.

10. Utilisation d'un peptide ayant la séquence d'acides aminés de SEQ. I.D. n° 10 pour la préparation d'un médicament afin d'induire une réponse des lymphocytes cytotoxiques.

11. Utilisation d'un peptide ayant la séquence d'acides aminés de SEQ. I.D. n° 4 pour la préparation d'un médicament afin d'induire une réponse des lymphocytes cytotoxiques.

12. Utilisation d'un peptide ayant la séquence d'acides aminés de SEQ. I.D. n° 17 pour la préparation d'un médicament destiné à induire une réponse des lymphocytes cytotoxiques.

13. Composition vaccinale capable d'induire une réponse des lymphocytes T cytotoxiques, comprenant un peptide ayant la séquence d'acides aminés de SEQ. I.D. n° 10 et un véhicule acceptable sur le plan pharmaceutique.

14. Composition vaccinale capable d'induire une réponse des lymphocytes T cytotoxiques, comprenant un peptide ayant la séquence d'acides aminés de SEQ. I.D. n° 4 et un véhicule acceptable sur le plan pharmaceutique.

15. Composition vaccinale capable d'induire une réponse des lymphocytes T cytotoxiques, comprenant un peptide ayant la séquence d'acides aminés de SEQ. I.D. n° 17 et un véhicule acceptable sur le plan pharmaceutique.

16. Composition pharmaceutique capable d'induire une réponse des lymphocytes T cytotoxiques, comprenant un peptide ayant la séquence d'acides aminés de SEQ. I.D. n° 10 et un véhicule acceptable sur le plan pharmaceutique.

17. Composition pharmaceutique capable d'induire une réponse des lymphocytes T cytotoxiques, comprenant un peptide ayant la séquence d'acides aminés de SEQ. I.D. n° 4 et un véhicule acceptable sur le plan pharmaceutique.

18. Composition pharmaceutique capable d'induire une réponse des lymphocytes T cytotoxiques, comprenant un peptide ayant la séquence d'acides aminés de SEQ. I.D. n° 17 et un véhicule acceptable sur le plan pharmaceutique.

19. Utilisation d'un peptide ayant la séquence d'acides aminés de SEQ. I.D. n°10 et un véhicule acceptable sur le plan pharmaceutique pour la préparation d'une composition vaccinale capable d'induire une réponse des lymphocytes T cytotoxiques destinée à une utilisation dans la prévention des infections par le virus de l'hépatite C.

20. Utilisation d'un peptide ayant la séquence d'acides aminés de SEQ. I.D. n° 4 et un véhicule acceptable sur le plan pharmaceutique pour la préparation d'une composition vaccinale capable d'induire une réponse des lymphocytes T cytotoxiques destinée à une utilisation dans la prévention des infections par le virus de l'hépatite C.

21. Utilisation d'un peptide ayant la séquence d'acides aminés de SEQ. I.D. n° 17 et un véhicule acceptable sur le plan pharmaceutique pour la préparation d'une composition vaccinale capable d'induire une réponse des lymphocytes T cytotoxiques destinée à une utilisation dans la prévention des infections par le virus de l'hépatite C.


### SEQUENCE LISTING

(1) GENERAL INFORMATION:
   (i) APPLICANT:
      (A) NAME: Gov't. of the United States as represented by the Department of Health and Human Services/National Institutes of Health
      (B) STREET: Box OTT
      (C) CITY: Bethesda
      (D) STATE OR PROVINCE: Maryland
      (E) COUNTRY: United States of America
      (F) POSTAL CODE: 20892
   (ii) TITLE OF INVENTION: Hepatitis C Virus Core Peptide For Stimulation of Cytotoxic T Lymphocytes and Diagnosis of HCV Exposure
   (iii) NUMBER OF SEQUENCES: 26
   (iv) CORRESPONDENCE ADDRESS:
      (A) ADDRESSEE: Birch, Stewart, Kolasch & Birch, LLP
      (B) STREET: P.O. Box 747
      (C) CITY: Falls Church
      (D) STATE: Virginia
      (E) COUNTRY: USA
      (F) ZIP: 22040-0747
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: US 08/224,978
      (B) FILING DATE: 08-APR-1994
      (C) CLASSIFICATION:
   (viii) ATTORNEY/AGENT INFORMATION:
      (A) NAME: Svensson, Leonard R.
      (B) REGISTRATION NUMBER: 30330
      (C) REFERENCE/DOCKET NUMBER: 1173-456P
   (ix) TELECOMMUNICATION INFORMATION:
      (A) TELEPHONE: 703-205-8000
      (B) TELEFAX: 703-205-8050
(2) INFORMATION FOR SEQ ID NO:1:
   (i) SEQUENCE CHARACTERISTICS:
      (A) LENGTH: 43 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:1:
   Gly Leu Tyr Pro His Glu Ala Leu Ala Ala Ser Pro Leu Glu Met Glu
   1               5                   10                  15
   Thr Gly Leu Tyr Thr Tyr Arg Ile Leu Glu Pro Arg Leu Glu Val Ala
               20                  25                  30
   Leu Gly Leu Tyr Ala Leu Ala Pro Arg Leu Glu
           35                  40
(2) INFORMATION FOR SEQ ID NO:2:
   (i) SEQUENCE CHARACTERISTICS:
      (A) LENGTH: 10 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:2:
   Gly Tyr Ile Pro Leu Val Gly Ala Pro Leu
   1               5                   10
(2) INFORMATION FOR SEQ ID NO:3:
   (i) SEQUENCE CHARACTERISTICS:
      (A) LENGTH: 10 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:3:
   Met Gly Tyr Ile Pro Leu Val Gly Ala Pro
   1               5                   10
(2) INFORMATION FOR SEQ ID NO:4:
   (i) SEQUENCE CHARACTERISTICS:
      (A) LENGTH: 10 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:4:
   Leu Met Gly Tyr Ile Pro Leu Val Gly Ala
   1               5                   10
(2) INFORMATION FOR SEQ ID NO:5:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:5:
   Asp Leu Met Gly Tyr Ile Pro Leu Val Gly
   1               5                   10
(2) INFORMATION FOR SEQ ID NO:6:
   (i) SEQUENCE CHARACTERISTICS:
      (A) LENGTH: 10 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:6:
   Ala Asp Leu Met Gly Tyr Ile Pro Leu Val
   1               5                   10
(2) INFORMATION FOR SEQ ID NO:7:
   (i) SEQUENCE CHARACTERISTICS:
      (A) LENGTH: 10 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:7:
   Phe Ala Asp Leu Met Gly Tyr Ile Pro Leu
   1               5                   10
(2) INFORMATION FOR SEQ ID NO:8:
   (i) SEQUENCE CHARACTERISTICS:
      (A) LENGTH: 10 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (xi) SEQUENCE DESCRIPTION: SEQ ID NO:8:
   Gly Phe Ala Asp Leu Met Gly Tyr Ile Pro
   1               5                   10
(2) INFORMATION FOR SEQ ID NO:9:
   (i) SEQUENCE CHARACTERISTICS:
      (A) LENGTH: 10 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Mus musculus
      (B) STRAIN: H-2d
   (ix) FEATURE:
      (A) NAME/KEY: Modified-site
      (B) LOCATION: 4..5
      (D) OTHER INFORMATION: /note= "may be Lys, Arg or His"
   (ix) FEATURE:
      (A) NAME/KEY: Modified-site
      (B) LOCATION: 8..9
      (D) OTHER INFORMATION: /note= "optional amino acid"
   (ix) FEATURE:
      (A) NAME/KEY: Modified-site
      (B) LOCATION: 9..10
      (D) OTHER INFORMATION: /note= "May be Leu, Ile or Phe"
   (xi) SEQUENCE DESCRIPTION: SEQ ID NO:9:
   Xaa Gly Pro Xaa Xaa Xaa Xaa Xaa Xaa Xaa
   1               5                   10
(2) INFORMATION FOR SEQ ID NO:10:
   (i) SEQUENCE CHARACTERISTICS:
      (A) LENGTH: 9 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..9
      (D) OTHER INFORMATION: /note= "peptide C7A2, see Fig. 1"
   (xi) SEQUENCE DESCRIPTION: SEQ ID NO:10:
   Asp Leu Met Gly Tyr Ile Pro Leu Val
   1               5
(2) INFORMATION FOR SEQ ID NO:11:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C1, see Fig. 1"
   (xi) SEQUENCE DESCRIPTION: SEQ ID NO:11:
   Arg Lys Thr Lys Arg Asn Thr Asn Arg Arg Pro Gln Asp Val Glu Phe
   1               5                   10                  15
(2) INFORMATION FOR SEQ ID NO:12:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C virus
      (B) STRAIN: H isolate
   (ix) FEATURE:
      (A) NAME/KEY: Peptide
      (B) LOCATION: 1..16
      (D) OTHER INFORMATION: /note= "peptide C2, see Fig. 1"
   (xi) SEQUENCE DESCRIPTION: SEQ ID NO:12:
   Gly Val Arg Ala Thr Arg Lys Thr Ser Glu Arg Ser Gln Pro Arg Gly
   1               5                   10                  15
(2) INFORMATION FOR SEQ ID NO:13:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C3, see Fig. 1"
   (xi) SEQUENCE DESCRIPTION: SEQ ID NO:13:
   Ala Arg Arg Pro Glu Gly Arg Thr Trp Ala Gln Pro Gly Tyr Pro Trp
   1               5                   10                  15
(2) INFORMATION FOR SEQ ID NO:14:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C4, see Fig. 1"
   (xi) SEQUENCE DESCRIPTION: SEQ ID NO:14:
   Ser Pro Arg Gly Ser Arg Pro Ser Trp Gly Pro Thr Asp Pro Arg Arg
   1               5                   10                  15
(2) INFORMATION FOR SEQ ID NO:15:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C5, see Fig. 1"
   (xi) SEQUENCE DESCRIPTION: SEQ ID NO:15:
   Asp Pro Arg Arg Arg Ser Arg Asn Leu Gly Lys Val Ile Asp Thr Leu
   1               5                   10                  15
(2) INFORMATION FOR SEQ ID NO:16:
   (i) SEQUENCE CHARACTERISTICS:
      (A) LENGTH: 17 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..17
      (D) OTHER INFORMATION: /note= "peptide c6, see Fig. 1"
   (xi) SEQUENCE DESCRIPTION: SEQ ID NO:16:
   Arg Asn Leu Gly Lys Val Ile Asp Thr Leu Thr Cys Gly Phe Ala Asp
   1               5                   10                  15
   Leu
(2) INFORMATION FOR SEQ ID NO:17:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C7, see Fig. 1"
   (xi) SEQUENCE DESCRIPTION: SEQ ID NO:17:
   Gly Phe Ala Asp Leu Met Gly Tyr Ile Pro Leu Val Gly Ala Pro Leu
   1               5                   10                  15
(2) INFORMATION FOR SEQ ID NO:18:
   (i) SEQUENCE CHARACTERISTICS:
      (A) LENGTH: 17 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis-C virus
      (B) STRAIN: H isolate
   (ix) FEATURE:
      (A) NAME/KEY: Peptide
      (B) LOCATION: 1..17
      (D) OTHER INFORMATION: /note= "peptide C8, see Fig. 1"
   (xi) SEQUENCE DESCRIPTION: SEQ ID NO:18:
   Val Gly Ala Pro Leu Gly Gly Ala Ala Arg Ala Leu Ala His Gly Val
   1               5                   10                  15
   Arg
(2) INFORMATION FOR SEQ ID NO:19:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C9, see Fig. 1"
   (xi) SEQUENCE DESCRIPTION: SEQ ID NO:19:
   His Gly Val Arg Val Leu Glu Asp Gly Val Asn Tyr Ala Thr Gly Asn
   1               5                   10                  15
(2) INFORMATION FOR SEQ ID NO:20:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C10, see Fig. 1"
   (xi) SEQUENCE DESCRIPTION: SEQ ID NO:20:
   Val Asn Tyr Ala Thr Gly Asn Leu Pro Gly Cys Ser Phe Ser Ile Phe
   1               5                   10                  15
(2) INFORMATION FOR SEQ ID NO:21:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (v) FRAGMENT TYPE: internal
   (vi) ORIGINAL SOURCE:
      (A) ORGANISM: Hepatitis C Virus
      (B) STRAIN: H isolate
   (ix) FEATURE:
      (A) NAME/KEY: Peptide
      (B) LOCATION: 1..16
      (D) OTHER INFORMATION: /note= "peptide C11, see Fig. 1"
   (xi) SEQUENCE DESCRIPTION: SEQ ID NO:21:
   Ser Phe Ser Ile Phe Leu Leu Ala Leu Leu Ser Cys Leu Thr Val Pro
   1               5                   10                  15
(2) INFORMATION FOR SEQ ID NO:22:
   (i) SEQUENCE CHARACTERISTICS:
      (A) LENGTH: 200 amino acids
      (B) TYPE: amino acid
      (C) STRANDEDNESS: not relevant
      (D) TOPOLOGY: linear
   (ii) MOLECULE TYPE: protein
   (iii) HYPOTHETICAL: NO
   (v) FRAGMENT TYPE: N-terminal
   (vi) ORIGINAL SOURCE:
      (A) ORGANISM: Hepatitis C Virus
      (B) STRAIN: FDA
   (ix) FEATURE:
      (A) NAME/KEY: Protein
      (B) LOCATION: 1..200
      (D) OTHER INFORMATION: /note= "HCV core protein sequence, FDA isolate, see Fig. 5"
   (xi) SEQUENCE DESCRIPTION: SEQ ID NO:22:
   Met Ser Thr Asn Pro Lys Pro Gln Arg Lys Thr Lys Arg Asn Thr Asn
   1               5                   10                  15
   Arg Arg Pro Gln Asp Val Glu Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Glu Met Arg Val Ala Gly Gly Arg Asp Gly
                   85                  90                  95
   Ser Cys Leu Pro Val Ala Leu Gly Glx Trp Gly Pro Thr Asp Pro Arg
               100                 105                 110
   Val Gly Arg Ala Ile Trp Val Arg Ser Ser Ile Pro Leu Arg Ala Val
           115                 120                 125
   Arg Arg Pro His Gly Val His Thr Ala Arg Arg Arg Pro Ser Trp Arg
       130                 135                 140
   Arg Asp Arg Ala Leu Ala His Gly Val Arg Val Leu Glu Asp Gly Val
   145                 150                 155                 160
   Asn Tyr Ala Thr Gly Asn Leu Pro Gly Cys Ser Phe Ser Ile Phe Leu
                   165                 170                 175
   Leu Ala Leu Leu Ser Cys Leu Thr Val Pro Ala Ser Ala Tyr Gln Val
               180                 185                 190
   Arg Asn Ser Ser Gly Leu Tyr His
           195                 200
(2) INFORMATION FOR SEQ ID NO:23:
   (i) SEQUENCE CHARACTERISTICS:
      (A) LENGTH: 200 amino acids
      (B) TYPE: amino acid
      (C) STRANDEDNESS: not relevant
      (D) TOPOLOGY: linear
   (ii) MOLECULE TYPE: protein
   (iii) HYPOTHETICAL: NO
   (v) FRAGMENT TYPE: N-terminal
   (vi) ORIGINAL SOURCE:
      (A) ORGANISM: Hepatitis C Virus
      (B) STRAIN: NYBC
   (ix) FEATURE:
      (A) NAME/KEY: Protein
      (B) LOCATION: 1..200
      (D) OTHER INFORMATION: /note= "HCV core protein, NYBC isolate, see Fig. 5"
   (xi) SEQUENCE DESCRIPTION: SEQ ID NO:23:
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu
           195                 200
(2) INFORMATION FOR SEQ ID NO:24:
   (i) SEQUENCE CHARACTERISTICS:
      (A) LENGTH: 192 amino acids
      (B) TYPE: amino acid
      (C) STRANDEDNESS: not relevant
      (D) TOPOLOGY: linear
   (ii) MOLECULE TYPE: protein
   (iii) HYPOTHETICAL: NO
   (v) FRAGMENT TYPE: N-terminal
   (vi) ORIGINAL SOURCE:
      (A) ORGANISM: Hepatitis C Virus
      (B) STRAIN: Chiron
   (ix) FEATURE:
      (A) NAME/KEY: Protein
      (B) LOCATION: 1..192
      (D) OTHER INFORMATION: /note= "HCV core protein, Chiron isolate, see Fig. 5"
   (xi) SEQUENCE DESCRIPTION: SEQ ID NO:24:
   Met Ser Thr Ile Pro Lys Pro Glu Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Thr Arg Lys Thr Ser Glu Arg Ser Gln Pro Arg Gly Arg Arg Gln Pro
       50                  55                  60
   Ile Pro Lys Val Arg Arg Pro Glu Gly Arg Thr Trp Ala Glu Pro Gly
   65                  70                  75                  80
   Tyr Pro Trp Pro Leu Tyr Gly Asn Glu Gly Cys Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
(2) INFORMATION FOR SEQ ID NO:25:
   (i) SEQUENCE CHARACTERISTICS:
      (A) LENGTH: 200 amino acids
      (B) TYPE: amino acid
      (C) STRANDEDNESS: not relevant
      (D) TOPOLOGY: linear
   (ii) MOLECULE TYPE: protein
   (iii) HYPOTHETICAL: NO
   (v) FRAGMENT TYPE: N-terminal
   (vi) ORIGINAL SOURCE:
      (A) ORGANISM: Hepatitis C Virus
      (B) STRAIN: Okayama
   (ix) FEATURE:
      (A) NAME/KEY: Protein
      (B) LOCATION: 1..200
      (D) OTHER INFORMATION: /note= "HCV core protein, Okayama isolate, see Fig. 5"
   (xi) SEQUENCE DESCRIPTION: SEQ ID NO:25:
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Val Tyr Leu Leu Pro Arg Arg Gly Pro Arg Leu Gly Val Arg Ala
           35                  40                  45
   Pro Arg Lys Thr Ser Glu Arg Ser Gln Pro Arg Gly Arg Arg Gln Pro
       50                  55                  60
   Ile Pro Lys Ala Arg Arg Pro Glu Gly Arg Thr Trp Ala Gln Pro Gly
   65                  70                  75                  80
   Tyr Pro Trp Pro Leu Tyr Gly Asn Glu Gly Leu Gly Trp Ala Gly Trp
                   85                  90                  95
   Leu Leu Ser Pro Arg Gly Ser Arg Pro Ser Trp Gly Pro Thr Asp Pro
               100                 105                 110
   Arg Arg Arg Ser Arg Asn Leu Gly Lys Val Ile Asp Thr Leu Thr Cys
           115                 120                 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Thr Pro Ala Ser Ala Tyr
               180                 185                 190
   Glu Val His Asn Val Ser Gly Ile
           195                 200
(2) INFORMATION FOR SEQ ID NO:26:
   (i) SEQUENCE CHARACTERISTICS:
      (A) LENGTH: 200 amino acids
      (B) TYPE: amino acid
      (C) STRANDEDNESS: not relevant
      (D) TOPOLOGY: linear
   (ii) MOLECULE TYPE: protein
   (iii) HYPOTHETICAL: NO
   (v) FRAGMENT TYPE: N-terminal
   (vi) ORIGINAL SOURCE:
      (A) ORGANISM: Hepatitis C Virus
      (B) STRAIN: Kato
   (ix) FEATURE:
      (A) NAME/KEY: Protein
      (B) LOCATION: 1..200
      (D) OTHER INFORMATION: /note= "HCV core protein, Kato isolate, see Fig. 5"
   (xi) SEQUENCE DESCRIPTION: SEQ ID NO:26:
   Met Ser Thr Asn Pro Lys Pro Gln Arg Lys Thr Lys Arg Asn Thr Asn
   1               5                   10                  15
   Arg Arg Pro Gln Asp Val Lys Phe Pro Gly Gly Gly Gln Ile Val Gly
               20                  25                  30
   Gly Gly Leu Tyr Leu Leu Pro Arg Arg Gly Pro Arg Leu Gly Val Arg
           35                  40                  45
   Ala Thr Arg Lys Thr Ser Glu Arg Ser Gln Pro Arg Gly Arg Arg Gln
       50                  55                  60
   Pro Ile Pro Lys Ala Arg Arg Pro Glu Gly Arg Thr Trp Ala Gln Pro
   65                  70                  75                  80
   Gly Tyr Pro Trp Pro Leu Tyr Gly Asn Glu Gly Met Gly Trp Ala Gly
                   85                  90                  95
   Trp Leu Leu Ser Pro Arg Gly Ser Arg Pro Ser Trp Gly Pro Thr Asp
               100                 105                 110
   Pro Arg Arg Arg Ser Arg Asn Leu Gly Lys Val Ile Asp Thr Leu Thr
           115                 120                 125
   Cys Gly Phe Ala Asp Leu Met Gly Tyr Ile Pro Leu Val Gly Ala Pro
       130                 135                 140
   Leu Gly Gly Ala Ala Arg Ala Leu Ala His Gly Val Arg Val Leu Glu
   145                 150                 155                 160
   Asp Gly Val Asn Tyr Ala Thr Gly Asn Leu Pro Gly Cys Ser Phe Ser
                   165                 170                 175
   Ile Phe Leu Leu Ala Leu Cys Leu Val Glx Pro Ser Gln Leu Pro Leu
               180                 185                 190
   Thr Arg Cys Ala Gln Arg Val Arg
           195                 200